(19) Europäisches Patentamt
European Patent Office
Office européen des brevets

(11) EP 2 698 155 A1

(12) EUROPEAN PATENT APPLICATION

(43) Date of publication:
**19.02.2014 Bulletin 2014/08**

(21) Application number: **12005899.5**

(22) Date of filing: **16.08.2012**

(51) Int Cl.:
***A61K 31/19*** *(2006.01)* ***A61K 9/00*** *(2006.01)*
***A61P 43/00*** *(2006.01)* ***A61P 35/00*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(71) Applicant: **Lunamed AG**
**7000 Chur (CH)**

(72) Inventors:
- **Truog, Peter**
  **7000 Chur (CH)**
- **Buschmann, Helmut H.**
  **52076 Aachen (Walheim) (DE)**

(74) Representative: **Peters, Hajo et al**
**ZACCO GmbH**
**Bayerstrasse 83**
**80335 München (DE)**

(54) **Pharmaceutical unit dosage form comprising 4-phenylbutyric acid**

(57) The invention provides a pharmaceutical unit dosage form comprising 4-phenylbutyric acid (4-PB) as a free acid or as a salt; as a prodrug, a codrug, a co-crystal or any salt or hydrate or solvate thereof. The invention further provides the use of said pharmaceutical dosage form for the prevention or treatment of cancer and methods for preparing said pharmaceutical dosage form.

EP 2 698 155 A1

## Description

### Field of the invention

The present invention refers to a pharmaceutical unit dosage form comprising 4-phenylbutyric acid (4-PB) as a free acid, a prodrug, codrug, co-crystal or a salt, solvate or hydrate thereof. In a further aspect the invention relates to the use of said pharmaceutical dosage form for the prevention or treatment of cancer.

### Background of the invention

Sodium phenylbutyric acid (4-PB) is a low molecular weight aromatic carboxylic acid. In 1987, it was approved for use in the United States for the treatment of urea cycle disorders in children (Brusilow SW, Maestri NE. "Urea cycle disorders: Diagnosis, pathophysiology, and therapy" Adv. Pediatr. (1996) 43:127-170). 4-PBA functions as an ammonia scavenger in the treatment of urea cycle disorders, clearing the toxic substance from the body. It is classified as an Orphan Drug, and manufactured in a 500mg tablet form (Buphenyl®; Medicis).

4-PBA has also been studied as a potential treatment for a variety of other disorders, such as cancer, or motor neuron disorders (for a review see lannitti and Palmieri, 2011, Drugs R D, 11(3): 227 - 249).

The clinical use of 4-phenylbutyric acid sodium salt has been hampered by the compound's short physiological half-life. 4-PBA breaks down quickly within the body to 4-phenylacetic acid and is rapidly eliminated from cells and excreted. As a result, very high concentrations are required in order to achieve therapeutic effects (e.g., up to 50 grams a day). Therapeutic use of such large amounts of 4-PBA is problematic for several reasons, including high cost, the need to continue therapy for months or years, and patient compliance issue (i.e., 50 grams is equal to 100 tablets per day).

It is an object of the present invention to provide new pharmaceutical unit dosage forms for 4-phenylbutyrate and methods of using said dosage form for medical purposes, especially for the prevention and treatment of cancer.

It has now been found that the pharmaceutical unit dosage form comprising 4-phenylbutyric acid according to the invention provides a therapeutically effective and safe treatment option.

Accordingly, the invention is drawn to a pharmaceutical unit dosage form comprising 4-phenylbutyric acid (4-PB) as a free acid or a salt; as a prodrug, a codrug, a co-crystal or any salt or hydrate or solvate thereof; and for the use of said dosage form in the prevention or treatment of cancer.

Phenylbutyric acid, especially 4-phenylbutyric acid as depicted in formula (I) is a histone deacetylase inhibitor that is known as a pharmaceutically active component for many years.

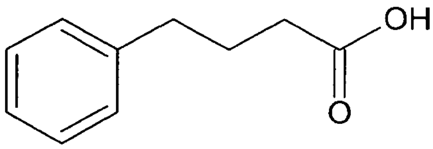

(I)

In form of its sodium salt it is marketed as a drug in the USA and European Union. Sodium phenylbutyrate is an orphan drug, marketed by Ucyclyd Pharma (Hunt Valley, USA) under the trade name Buphenyl and by Swedish Orphan International (Sweden) as Ammonaps for the treatment of urea cycle disorders.

### DETAILED DESCRIPTION OF THE INVENTION

The present invention refers to 4-phenylbutyric acid, pharmaceutical unit dosage forms comprising 4-phenylbutyric acid and their medical uses.

Prior to describing exemplary embodiments of the present invention, definitions important for understanding the present invention are given as follows.

As used in this specification and in the appended claims, the singular forms of "a" and "an" also include the respective plurals unless the context clearly dictates otherwise.

In the context of the present invention, the terms "about" and "approximately" denote an interval of accuracy that a person skilled in the art will understand to still ensure the technical effect of the feature in question. The term typically indicates a deviation from the indicated numerical value of ±20 %, preferably ±15 %, more preferably ±10 %, and even more preferably ±5 %.

It is to be understood that the term "comprising" is not limiting. For the purposes of the present invention the term "consisting of is considered to be a preferred embodiment of the term "comprising of. If hereinafter a group is defined to comprise at least a certain number of embodiments, this is meant to also encompass a group which preferably consists of these embodiments only.

Furthermore, the terms "first", "second", "third" or "(a)", "(b)", "(c)", "(d)" etc. and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

A first aspect of the present invention refers to 4-phenylbutyric acid, pharmaceutical unit dosage forms comprising 4-phenylbutyric acid and derivatives such as codrugs and prodrugs thereof and their medical uses. Thus, explicitly encompassed in the scope of the present invention is 4-phenylbutyric acid as a free acid or as a salt or a hydrate or solvate thereof; in neutral form, as an isomer, as an ester; or any hydrate or solvate thereof. Therefore, for the purpose of the present invention, any mentioning of "4-phenylbutyric acid" (or abbreviated herein as "4-PB") refers also to the 4-phenylbutyric acid as a free acid or as a salt or a hydrate or solvate thereof; in neutral form, as an isomer, as an ester; or any hydrate or solvate thereof - also without explicit mentioning.

Along the same lines, the expression "compounds of the invention" as used herein, refers to 4-PB as a free acid, a prodrug, a codrug, a co-crystal or as a salt or a hydrate or solvate thereof;, as an ester; or any hydrate or solvate thereof.

In a preferred embodiment the salt form of 4-PB is of the alkaline or earth alkaline type, e g. lithium, sodium, potassium, magnesium or calcium, and also ammonium salts are contemplated, as well as solvates and hydrates of these salts.

In a preferred embodiment 4-PB is in the form of a potassium salt or a solvate or hydrate thereof.
In a preferred embodiment 4-PB is in the form of a lithium salt or a solvate or hydrate thereof.

In a preferred embodiment 4-PB is in the form of a sodium salt or a solvate or hydrate thereof.

In a preferred embodiment 4-PB is in the form of a magnesium salt or a solvate or hydrate thereof.

Also part of the invention are any derivatives, such as prodrugs (e.g. an amide) or codrugs of 4-PB as defined herein or a solvate or hydrate thereof.

The present invention, in one aspect refers to a pharmaceutical unit dosage form comprising 4-PB as a free acid, a prodrug, a codrug, a co-crystal or as a salt or a hydrate or solvate thereof; in neutral form, as an isomer, as an ester; or any hydrate or solvate thereof.

The present invention, in another aspect refers to a pharmaceutical unit dosage form comprising 4-PB as a free acid, a prodrug, a codrug, a co-crystal or as a salt or a hydrate or solvate thereof; in neutral form, as an isomer, as an ester; or any hydrate or solvate thereof, for use as a medicament.

The present invention, in another aspect refers to a pharmaceutical unit dosage form comprising 4-PB as a free acid, a prodrug, a codrug, a co-crystal or as a salt or a hydrate or solvate thereof; in neutral form, as an isomer, as an ester; or any hydrate or solvate thereof, for use as an anti-cancer drug.

As used herein, the term "anticancer drug" refers to a drug that is used for the prevention or the treatment of cancer.

The present invention, in another aspect refers to a method of treatment of cancer, by providing to a patient in need thereof a sufficient amount of 4-PB as a free acid, a prodrug, a codrug, a co-crystal or as a salt or a hydrate or solvate thereof; in neutral form, as an isomer, as an ester; or any hydrate or solvate thereof.

The present invention, in one aspect refers to 4-PB as a free acid, a prodrug, a codrug, a co-crystal or as a salt or a hydrate or solvate thereof; in neutral form, as an isomer, as an ester; or any hydrate or solvate thereof, to be administered alone as part of a pharmaceutical unit dosage form.

## Prodrugs

In one aspect of the invention the pharmaceutical composition comprises a prodrug of 4-PB.

The term "prodrug" refers to compounds that are rapidly transformed in vivo to yield the phenylbutyric acid parent compound, for example by hydrolysis in blood. Functional groups that may be rapidly transformed, by metabolic cleavage, in vivo form a class of groups reactive with the carboxyl group of the compounds of this invention. They include, but are not limited to such groups as alkanoyl (such as acetyl, propanoyl, butanoyl, and the like), unsubstituted and substituted aroyl (such as benzoyl and substituted benzoyl), alkoxycarbonyl (such as ethoxycarbonyl), trialkylsilyl (such as trimethyl- and triethysilyl), monoesters formed with dicarboxylic acids (such as succinyl), and the like. Because of the ease with which the metabolically cleavable groups of the compounds of this invention are cleaved in vivo, the compounds bearing such groups act as pro-drugs. The compounds bearing the metabolically cleavable groups have the advantage that they may exhibit improved bioavailability as a result of enhanced solubility and/or rate of absorption conferred upon the parent compound by virtue of the presence of the metabolically cleavable group. A thorough discussion is provided in Prodrugs and Targeted Delivery: Towards Better ADME Properties (Methods and Principles in Medicinal Chemistry), Mannhold et al, Ed., Wiley-VCH (2010), which is incorporated herein by reference.

In a preferred embodiment of the invention the prodrug is used in an oral application form whereby the release

profile of the prodrug from said orally given pharmaceutical composition is specifically adjusted to the cleavage characteristics of said prodrug to yield an improved release profile of 4-PB.

For example, a pharmaceutical composition with sustained release prevents a release in the upper GI tract (such as the stomach) and will release the drug in the lower GI tract (e.g. in the small intestine) where the slightly alkaline pH value will decrease the transfer of the deprotonated 4-PB through the intestine wall. If the sustained release form is combined with a more lipophilic prodrug of 4-PB the transfer of said prodrug in the lower GI tract is increased.

In a further embodiment of the invention, the pharmaceutical composition comprises 4-PB together with a derivative of 4-PB such as a prodrug or codrug of 4-PB. This would enable pharmaceutical composition with improved release characteristics. As an example, an immediate release formulation comprising the 4-PB together with a (preferably slowly metabolized) prodrug would exhibit release characteristics with a first immediate release of 4-PB and a further sustained release by subsequent cleavage of the immediately released prodrug.

In another embodiment the pharmaceutical composition comprises different prodrugs of 4-PB with different cleavage characteristics or different bioavailalibity in order to optimize the release profile of 4-PB.

In a preferred embodiment of the invention the prodrug is an ester, an amide or an anhydride of 4-PB.

In a preferred embodiment of the invention the 4-phenylbutyric acid prodrug is a carbonic acid anhydride, being preferably pivaloyloxymethyl-4-phenylbutyrate.

In another embodiment of the invention the prodrug is an ester of 4-phenylbutyric acid, which preferably is an ester with an alcohol selected from the group consisting of methanol, ethanol, trichloroethanol, propanol, n-butanol, isobutanol, 1-pentanol, 2-pentanol, 3-pentanol, 2-methyl-2-butanol, 2-methyl-1-butanol, 3-methy-1-butanol, 3-methyl-2-butanol, 2,2-dimethyl-1-propanol, 1-hexanol, 2-hexanol, 3-hexanol, 2-methyl-1-pentanol, 3-methyl-1-pentanol, 4-methyl-1-pentanol, 2-methyl-2-pentanol, 3-methyl-2-pentanol, 4-methyl-2-pentanol, 2-methyl-3-pentanol, 3-methyl-3-pentanol, 2,2-dimethyl-1-butanol, 2,3-dimethyl-1-butanol, 2,3-dimethyl-2-butanol, 3,3-dimethyl-2-butanol, 2-ethyl-1-butanol and 2-propyl-pentanol.

In a further embodiment of the invention the prodrug is an ester of 4-phenylbutyric acid with polyvalent alcohol, which is preferably a divalent, trivalent or tetravalent alcohol.

Non-limited examples for such polyvalent alcohols are ethylene glycol, 1,3-propanediol, 1,3-butanediol, 1,4-butanediol, glycerol and pentaerythritol.

In a specific embodiment of the invention the prodrug is an ester of 4-phenylbutyric acid with glycerol, which can be the mono-ester, the di-ester or the tri-ester.

In a further embodiment of the invention the prodrug is a diethylcarbonate prodrug of 4-phenylbutyric acid.

In another embodiment of the invention the prodrug is an 4-phenylbutyric amide which is formed with an amine preferably selected from the group consisting of a naturally occurring amino acid such as alanine, arginine, asparagine, aspartic acid, cysteine, glutamine, glutamic acid, glycine, histidine, isoleucine, leucine, lysine, methionine, phenylalanine, proline, serine, threonine, tryptophan, tyrosine, and valine, being more preferably phenylalanine.

In a preferred embodiment the 4-phenylbutyrate prodrug is butyroyloxymethyl-4-phenylbutyrate.

Codrugs

In another aspect of the invention the pharmaceutical composition comprises a codrug of 4-PB.

The term "codrug" as used herein means a compound, or a prodrug form thereof, comprising a first residue of phenylbutyric acid associated with a second residue, wherein both residues, in their unlinked forms (e.g., in the absence of the association), are biologically active. In preferred embodiments, one the second residue is a small molecule. The association between said residues is covalent and is either direct or indirect through a linker. The first residue can be the same or different from the second. The codrugs referred to herein may optionally be homocodrugs or heterocodrugs. A "homocodrug," also termed a "symmetrical codrug," refers to a codrug that produces, upon cleavage or dissociation, two or more molecules of phenylbutyric acid, and no other drug molecules, i.e., the homo codrug is composed primarily of two or more residues of a phenylbutyric acid, without incorporating a residue of a second drug. A "heterocodrug," also termed an "asymmetrical codrug," refers to a codrug that produces, upon cleavage or dissociation, residues of at least two different drugs.

In a preferred embodiment said codrug comprises:

a) at least two components, including 4-PB as a first component and a second component B selected from the group consisting of HDAC inhibitors different from phenylbutyric acid, short chain fatty acids, NSAIDs, polyphenols, stilbenes, flavones and flavinoids, vitamin D derivatives, curcumin derivatives, organosulfur compounds, organoselenium compounds, polyunsaturated fatty acids and esters thereof, carotenoids, chalcone derivatives, steroids, cyclic triterpenes, phenylpropene derivatives, monoterpenes and chemopreventive phytochemicals;

b) a linkage covalently linking said at least two components to form said codrug, said linkage is cleaved under

physiological conditions after the codrug has been administered to the subject to regenerate said components.

Yet another aspect of the invention provides a pharmaceutical composition comprising a codrug, wherein the codrug has the following structural formula:

$$R_1 - L- (R_2)n$$

wherein the first component is $R_1$;
the linker L
the second component is $R_2$;
$R_1$ represents, independently, a residue of a 4-phenylbutyric acid including 4-phenylbutyrate, a 4-phenylbutamido group, or a 4-phenylbutoxy group;
$R_2$ represents a residue of a compound B selected from short chain fatty acids, HDAC inhibitors different from phenylbutyric acid, NSAIDs, polyphenols, stilbenes, flavones and flavinoids, vitamin D derivatives, curcumin derivatives, organosulfur compounds, organoselenium compounds, polyunsaturated fatty acids and esters thereof, carotenoids, chalcone derivatives, steroids, cyclic triterpenes, phenylpropene derivatives, monoterpenes and chemopreventive phytochemicals;
n is an integer of from 1 to 4;
and L is selected from a direct bond and a linking group.

The linker L may be either a direct bond between individual components, or it may include a linking group. The first and second components of the codrugs of the present invention may be linked via reversible covalent bonds such as ester, amide, carbamate, carbonate, cyclic ketal, thioester, thioamide, thiocarbamate, thiocarbonate, xanthate and phosphate ester bonds, so that, at the required site in the body, they are cleaved to regenerate the active forms of the constituent pharmaceutically active agents.

In the following non-limited examples for compound B selected from short chain fatty acids, HDAC inhibitors different from phenylbutyric acid, NSAIDs, polyphenols, stilbenes, flavones and flavinoids, vitamin D derivatives, curcumin derivatives, organosulfur compounds, organoselenium compounds, polyunsaturated fatty acids and esters thereof, carotenoids, chalcone derivatives, steroids, cyclic triterpenes, phenylpropene derivatives, monoterpenes and chemopreventive phytochemicals to generate a 4-PB codrug or a co-crystal (see following chapter) are given.

Non-limited examples of short chain fatty acids are propionic acid, n-butyric acid, valproic acid, AN-9 (pivaloyloxymethyl butyrate), 2-n-propyl-4-pentynoic acid, valproyl hydroxamic acid, N-(1-hydroxyethyl)-valpromide, 2-hydroxypropyl-valpromide and N-methoxy valpromide and alpha-lipoic acid.

Non-limited examples of HDAC inhibitors not related to phenylbutyric acid for use as component B are voriostat (SAHA), belinostat (PXD 101), resminostat (4SC-201), panobinostat (LBH 589), CHR-2845, SB 939, givinostat (ITF 2357), PCI 24781, PCI-34051, JNJ 26481585, CUDC 101, oxamflatin, carboxy cinnamic acid bishydroxamic acid (CBHA), pyroxamide, scriptaid, trichostatin A (TSA), NVP-LAQ824, etinostat (SNDX-275, MS-275), mocetinostat (MGDC 0103), chidamide (CS 055/HBI 8000), N-acetyl dinaline (CI-994), MS-275, chlamydocin, romidepsin, trapoxin A, trapoxin B, apicidin, depsipeptide (FK-228, FR901228), TPX-HA analogue (CHAP), trapoxin, R306465, suberic bishydroxamic acid (SBHA), azelaic bishydroxamic acid (ABHA), SK-7041, SK-7068, CG-1521, tubacin, MS-275, trifluoromethylketone, depudecine, MGCD-0103, romidepsin (Istodax) and MS-27-275.

Non-limited examples of NSAIDs for use as component B are acetylsalicylic acid, para-aminosalicylic acid ethenzamide, salicylamide, diflunisal, salsol, salsalate, salicin, methylsalicylate, trisalicylates, meclofenamic acid, niflumic acid, flufenamic acid, mefenamic acid, etofenamate, tolfenamic acid, bufexamac, diclofenac, lonazolac, acemetacin, indomethacin, tolmetin, nalbumetone, sulindac, sulindac sulphide, ibuprofen, flurbiprofen, ketoprofen, fenoprofen, fenbufen, tiaprofenic acid, ketorolac, etodolac, bromfenac, metamizil, propyphenazone, paracetamol, phenidine, propacetamol, kebuzone, mofebutazone, oxyphenbutazone, phenylbutazone, naproxen, tolmetin, oxaprosin, piroxicam, tenoxicam, isoxicam, lornoxicam, nimesulide, NS-398, flosulid, L-745337, rofecoxib, celecoxib, etoricoxib, parecoxib, lumiracoxib, valdecoxib. For further examples see Christoph and Buschmann, 2002 (Cyclogenase inhibition: From NSAIDs to selective COX-2 inhibitors. In: Sundermann B, Buschmann H; Christoph T; Friderichs E; Maul C (Eds.): Analgesics: From Chemistry and Pharmacology to Clinical Application, page 13-126, Weinheim: Wiley-VCH 2002) which is incorporated herein by reference.

Non-limited examples of polyphenols are (-)-epicatechin (EC), (-)-epicatechin-3-gallate (ECG), (-)-epigallocatechin-3-gallate (EGCG), (-)-epigallocatechin (EGC), (+)-catechin" (*)-gallocatechin, (-)-robinetinidol, (+)-fisetinidol, (-)-fisetinidol, (+)-afzelechin, (+)-epiafzelechin and (-)-epiafzelechin; SU11274, theacetrin, theaflavin, theasinesin, theanaphthoquinone, olongtheanin, elagic acid, punigalacin, pinoresinol, lariciresinol, secoisolariciresinol, matairesinol, hydroxymatairesinol, syringaresinol and sesamin.

Non-limited examples of stilbenes are resveratrol, resveratrol-triacetate, digalloylresveratrol, pterostilbene, hydroxystilbene, dihydroxystilbene, trihydroxystilbene, tetrahydroxystilbene, pentahydroxystilbene, trimethoxystilbene, tetramethoxystilbene, pentamethoxystilbene, KITC, N-hydroxy-N'-(trimethoxphenyl)-trimethoxy-benzamidine, gloriosaol A, gloriasol B, gloriasol C, luteolin and piceatannol. For further examples see Fulda 2010 (Drug Discovery Today 15: 1590-1611) which is incorporated herein by reference.

Non-limited examples of flavones and flavinoids are quercetin, apigenin, biapigenin, phenoxodiol (NV-128), triphendiol, NV-143 (ME-143), naringenin, naringin, narirutin, hesperetin, hesperidin, daidzein, dihydro-daidzein, glycitein, genistein, dihydro-genistein, O-DMA, myricetin, campherol, baicalin, pelargonidin, cyanidin, dephinidin, 4-bromoflavone.

Non-limited examples of vitamin D and vitamin D derivatives are vitamin D2, $1\alpha,25(OH)_2D_3$, 22-oxa-$1\alpha,25(OH)_2D_3$ (OCT), 19-nor-$1\alpha,25(OH)_2D_2$, $1\alpha(OH)_2D_3$, calcipotriol, $1\alpha,25(OH)_2$-16-ene-23-yne-$D_3$, KH1060, EB1089, MC1288, ED-71, 2MbisP and Gemini analogs of vitamin D3 such as BXL 01-0108, BXL 01-0101, BXL 01-0098, BXL 01-0120, BXL 01-0117, BXL 01-0114, Gemini 2198, Gemini 3582, Gemini 0084, Gemini 0088, Gemini 0072 and Gemini 0097. For further examples see Brown et al. 2008 (Mol Aspects Medicine 29: 433-452) which is incorporated herein by reference.

Non-limited examples of curcumin derivatives are curcumin, tetrahydrocurcumin, dihydrocurcumin, hexahydrocurcumin, hexahydrocurcuminol, curcuminglucucronide, demethoxycurcumin, bisdemethoxycurcumin, cyclocurcumin, curcumin sulphate, cassumuin A, cassumuin B, [6]-paradol, oregonin, yakuchinone A, chlorogenic acid, ferulic acid, [6]-gingerol, dehydrozingerone, For further examples see Anand et al. 2008 (Biochem. Pharmacol. 76: 1590-1611) which is incorporated herein by reference.

Non-limited examples of organosulfur compounds are diallyldisulfide, diallylsulfide, 1-isothiocyanato-4-methylsulfinylbutane (sulphoraphane), allicin, benzyl isothiocyanate, alliin, oltipraz, anethole dithiolethione (ADT), 6-HITC, anethole trithione, 1,2-dithiole-3-thione, $\gamma$-glutamyl-S-allylcysteine, $\gamma$-glutamyl-S-trans-1-propenylcysteine, ajoene, S-allyl cysteine, S-allylmercapto cysteine, phenyl isothiocyanate and diallyl trisulfide.

Non-limited examples of organoselenium compounds are benzyl seleno cyanide, benzyl selenocyanate (BSC), and 1,4-phenylene bis(methylene) selenocyanate.

Non-limited examples of polyunsaturated fatty acids and esters thereof are $\omega$-3 or $\omega$-6 polyunsuturated fatty acids (PUFAs) such as $\alpha$-linolenic acid, docosahexaenoic acid (DHA), DHA ethyl ester, hexadecatrienoic acid (HTA), stearidonic acid, eicosatetraenoic acid (ETA), eicosapentaenoic acid (EPA), heneicosapentaenoic acid (HPA), docosapentaenoic acid (DPA), tetracosapentaenoic acid, tetracosahexaenoic acid and eicosapentaenoic acid.

Non-limited examples of carotenoids are zeaxanthin, $\beta$-carotene, lutein, $\beta$-cryptoxanthine, lycopene, lycopersene, phytofluene, hexahydrolycopene, torulene, $\alpha$-zeacarotene, alloxanthin, cynthiaxanthin, pectenoxanthin, cryptomonaxanthin, crustaxanthin, gazaniaxanthin, OH-chlorobactene, loroxanthin $\beta$, lycoxanthin $\gamma$, rhodopin, rhodopinol, and saproxanthin.

Non-limited examples of chalcone derivatives are chalcone, naringenin chalcone, phloridzin, myrigalone B, 2-hydroxychalcone, 4-hydroxychalcone, 2,4-dihydroxychalcone, 2-hydroxy-4-methoxychalcone, xanthohumol B, xanthohumol D, dihydroxyxanthohumol, isobavachalcon, robteine, buteine, licochalcone A, isoliquiritigenin, 1,2-dihydroparatocarpin A, isoliquiritin apioside, xanthohumol, broussochalcone A, phloretin, isobutrin, desmethylxanthohumol, asebogenin, isoliquiritigenin 2'-methyl ether, ON-III, licochalcone B, licochalcone D, cardamonin, flavokawain A, flavokawain B, hydroxysafflor yellow A, hydroxyderricin, uvaretin, isouvaretin, diuvaretin, isolespeol, licochalcone E, xanthoangelol and pedicine.

Non-limited examples of or steroids are diosgenin, oleandrin, oleanolic acid and guggulsterone,

Non-limited examples of cyclic triterpenes are glycyrrhizin, glycyrrhetinic acid, 18-$\beta$-glycyrrhetinic acid, bryonolic acid, betulinic acid, lupeol, celastrol, ursolic acid, acetyl-11-keto-$\beta$-boswellic acid (AKBA), $\alpha$-boswellic acid, $\beta$-boswellic acid, 11-keto-$\beta$-boswellic acid, ganoderic acid C, ganoderic acid D, ginsenoside Rh2, ginsenoside Rf, ginsenoside Rg3, dihidrocucurbitacin B, cucurbitacin R, cucurbitacin B, cucurbitacin A, cucurbitacin E, cucurbitacin D, cucurbitacin Q (picracin), cucurbitacin R diglycoside, demethylzeylasteral, wilforic acid A, 24-metyllanosta-8,24(31)diene-3$\beta$,22$\zeta$-diol, (22S,24R)24-metyllanosta-8-en-28-epoxy-3$\beta$,28$\zeta$-diol, (22R,24S)24-metyllanosta-8-en-28-epoxy-3$\beta$,28$\zeta$-diol, 17$\alpha$-23-(E)-Dammara-20,23-diene-3$\beta$,25-diol, 2,3-dihydroxy-1,3,5(10),7-tetraene-6$\alpha$(1'-hydroxy-ethyl)-24-nor-D,A-friedooleane-29-oic acid, pristimerin, tripterygone, oleanolic acod. 3-epi-katonic acid, triptotriterpenonic acid A, 2$\alpha$,3$\beta$-dihydroxy-olean-12-ene-22,29-lactone, kaikasaponin I, podocarpaside, soyasaponin III, astrasiversianin II, astrasiversianin I, astragaloside I, astragaloside II, astragaloside IV, astragaloside VI, cyclocanthoside E, cyclocanthoside G, avicin G, buddlejasaponin IV, momilactone B, carnosol, carnosic acid, impressic acid and lantanolic acid. For further examples see Rios et al. 2010 (J. Ethnopharmacol. 128: 1-14) which is incorporated herein by reference.

Non-limited examples of phenylpropene derivatives are 1'-acetoxychavicol acetate, eugenol, chavicol, safrole and estragole.

Non-limited examples of monoterpenes are limonene, pinenene, menthol, geraniol, camphene, sabinene, cadinine, perillyl alcohol (PA), geranial, neral, nerol, carvone, 1,8-cineol, carvacrol, thymol, and alpha-terpineol.

Non-limited examples for chemopreventive phytochemicals are yakuchinone A, yakuchinone B, capsaicin,

dibenzoylmethane, piperine, kahweol, grayanotoxan, kauran, atisan, giberellan, indiruibin-3'-monoxime, caffeic acid, caffeic acid phenethyl ester (CAPE), emodin, linalol, quinic acid, garcinol, sesamin, theaflavin-3,3'-digallate, sanguinarine, silymarin, mangostin, mangiferin, butein, berberine, glabridin, carnosol, β-lapachone, evodiamine, wogonin, tanshinones IIA, tanshinones I, carnosol, zerumbone, sulforaphane, phytic acid, ascorbic acid, anethole, indole 3-carbinol, thymoquinone and plumbagin.

Non-limited examples of vitamine E derivatives are a-tocotrienol, p-tocotrienol, γ-tocotrienol, δ-tocotrienol, chalcone, α-tocopherol, β-tocopherol, γ-tocopherol, δ-tocopherol, α-tocomoneol, β-tocomoneol, γ-tocomoneol, δ-tocomoneol, α-MDT, β-MDT, γ-MDT, δ-MDT, whereby the tocotrienol compounds are preferred.

The covalent bonds between residues include a bonding structure such as:

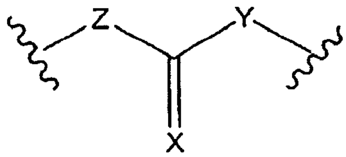

wherein Z is O, N, $-CH_2-$, $-CH_2-O-$ or $-CH_2-S-$, Y is O, or N, and X is or S.

In a further embodiment, the linker comprises an azo group, which in a preferred embodiment is a linking group according the following formula:

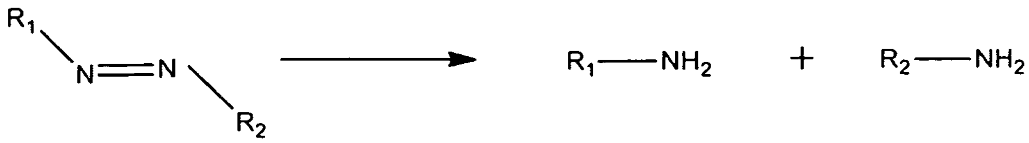

wherein $R_1$ and $R_2$ represent the above defined residues.

The azo group can be cleaved by the enzyme azoreductase to yield the respective amines.

In another embodiment, the linker comprises a carbonate ester moiety, which in a preferred embodiment is a linking group according the following formula:

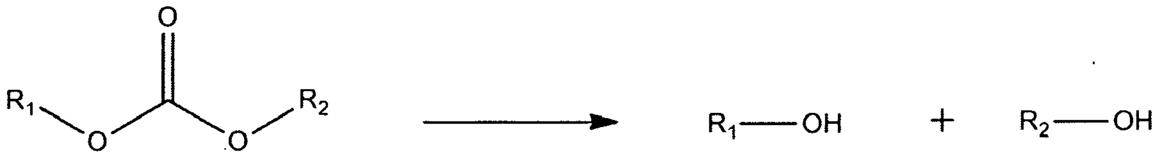

wherein $R_1$ and $R_2$ represent the above defined residues.

As shown above, the cleavage of the carbonate ester moiety yields two alcohol compounds.

In a further embodiment, the linker comprises a carbamate moiety, which in a preferred embodiment is a linking group according the following formula:

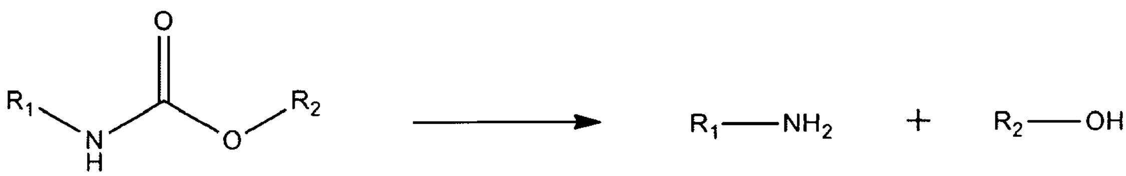

wherein $R_1$ and $R_2$ represent the above defined residues.

As shown above, the cleavage of the carbamate moiety yields an amine and an alcohol.

In another embodiment, the linker comprises a dicarboxylic acid ester moiety, which in a preferred embodiment is a linking group according the following formula:

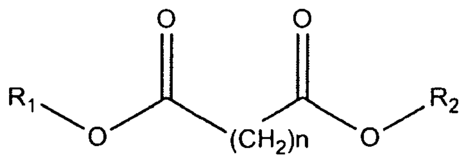

wherein $R_1$ and $R_2$ represent the above defined residues and n is from 1 to 12.

In a further embodiment, the linker comprises a linear dihydroxy residue, which in a preferred embodiment is a linking group according the following formula:

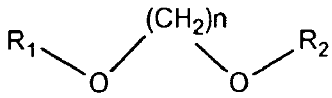

wherein $R_1$ and $R_2$ represent the above defined residues and n is from 1 to 12.

In one embodiment, the linker comprises a linear diamino moiety, which in a preferred embodiment is a linking group according the following formula:

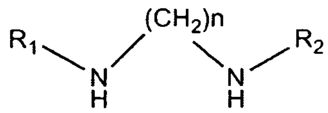

wherein $R_1$ and $R_2$ represent the above defined residues and n is from 1 to 12.

The rate of cleavage of the individual component residues can be controlled by the type of bond, the choice of constituent moieties, and the physical form of the codrug. The lability of the selected bond type may be enzyme-specific. In some embodiments according to the present invention, the bond is selectively labile in the presence of an esterase. In other embodiments of the invention, the bond is chemically labile, e.g., to acid- or base-catalyzed hydrolysis.

The physiologically labile linkage may be any linkage that is labile under conditions approximating those found in physiologic fluids, such as is found in the gastrointestinal tract, the blood, the liquor, the dermis or viable epidermis. The linkage may be a direct bond (for instance, ester, amide, carbamate, carbonate, cyclic ketal, thioester, thioamide, thiocarbamate, thiocarbonate, xanthate, phosphate ester, sulfonate, or a sulfamate linkage) or may be a linking group (for instance a $C_1$-$C_{12}$ dialcohol, a $C_1$-$C_{12}$ hydroxylalkanoic acid, a $C_1$-$C_{12}$ hydroxyalkylamine, a $C_1$-$C_{12}$ diacid, a $C_1$-$C_{12}$ amino acid, or a $C_1$-$C_{12}$ diamine). Especially preferred linkages are direct amide, ester, carbonate, carbamate, and sulfamate linkages, and linkages via succinic acid, salicylic acid, diglycolic acid, oxa acids, oxamethylene, and halides thereof. The linkages are labile under physiologic conditions, which generally mean pH of about 6 to about 8. The lability of the linkages depends upon the particular type of linkage, the precise pH and ionic strength of the physiologic fluid, and the presence or absence of enzymes that tend to catalyze hydrolysis reactions in vivo. In general, lability of the linkage in vivo is measured relative to the stability of the linkage when the codrug has not been solubilized in a physiologic fluid.

Thus, while some codrugs according to the present invention may be relatively stable in some physiologic fluids, nonetheless, they are relatively vulnerable to hydrolysis in vivo (or in vitro, when dissolved in physiologic fluids, whether naturally occurring or simulated) as compared to when they are neat or dissolved in non-physiologic fluids (e.g., non-aqueous solvents such as acetone).

Thus, the labile linkages are such that, when the codrug is dissolved in an aqueous solution, especially a physiologic fluid such as blood or liquor, the reaction is driven to the hydrolysis products, which include the components set forth above.

Codrugs according to the present invention may be synthesized in the manner as known for the skilled person. In general, where the first and second components are to be directly linked, the 4-phenylbutyric acid as first component is condensed with the second component B under conditions suitable for forming a linkage that is labile under physiologic conditions. In some cases it is necessary to block some reactive groups on one, the other, or both of the components. Where the two components are to be covalently linked via a linker, such as oxamethylene, succinic acid, or diglycolic acid, it is advantageous to first condense the 4-phenylbutyric acid with the linker. In some cases it is advantageous to perform the reaction in a suitable solvent, such as acetonitrile, in the presence of suitable catalysts, such as carbodiimides including EDCI (1-ethyl-3-(3-dimethylaminopropyl)-carbodiimide) and DCC (DCC: dicyclohexylcarbodiimide), or under conditions suitable to drive off water of condensation or other reaction products (e.g., reflux), or a combination of two or

more thereof. After the 4-phenylbutyric acid residue is condensed with the linker, the combined first component and linker may then be condensed with the second component B. Again, in some cases it is advantageous to perform the reaction in a suitable solvent, such as acetonitrile, in the presence of suitable catalysts, such as carbodiimides including EDCI and DCC, or under conditions suitable to drive off , water of condensation or other reaction products (e.g., reflux), or a combination of two or more thereof. Where one or more active groups have been blocked, it may be advantageous to remove the blocking groups under selective conditions, however it may also be advantageous, where the hydrolysis product of the blocking group and the blocked group is physiologically benign, to leave the active groups blocked.

The person having skill in the art will recognize that, while diacids, dialcohols, amino acids, etc. are described above as being suitable linkers, other linkers are contemplated as being within the present invention. For instance, while the hydrolysis product of a codrug according to the present invention may comprise a diacid, the actual reagent used to make the linkage may be, for example, an acylhalide such as succinyl chloride. The person having skill in the art will recognize that other possible acid, alcohol, amino, sulfato, and sulfamoyl derivatives may be used as reagents to make the corresponding linkage.

Where the first and second components are to be directly linked via a covalent bond, essentially the same process is conducted, except that in this case there is no need for a step of adding a linker. The 4-phenylbutyric acid residue and the second component B are merely combined under conditions suitable for forming the covalent bond. In some cases it may be desirable to block certain active groups on one, the other, or both of the pharmaceutically active moieties. In some cases it may be desirable to use a suitable solvent, such as acetonitrile, a catalyst suitable to form the direct bond, such as carbodiimides including EDCI and DCC, or conditions designed to drive off water of condensation (e.g., reflux) or other reaction by-products.

The person having skill in the art will recognize that, while in most cases the 4-phenylbutyric acid and the second component B may be directly linked in their original form, it is possible for the active groups to be derivatized to increase their reactivity. For instance, where the second moiety is an alcohol (i.e., has a free hydroxyl group), the 4-phenylbutyric acid may be derivatized to form the corresponding acid halide, such as an acid chloride or an acid bromide. The person having skill in the art will recognize that other possibilities exist for increasing yield, lowering production costs, improving purity, etc. of the codrug according to the present invention by using conventionally derivatized starting materials to make codrugs according to the present invention.

Exemplary reaction schemes according to the present invention are illustrated in Schemes 1-4, below. These Schemes can be generalized by substituting other components B having at least one functional group that can form a covalent bond to another therapeutic agent having a similar or different functional group, either directly or indirectly through a pharmaceutically acceptable linker. The person of skill in the art will appreciate that these schemes also may be generalized by using other appropriate linkers.

Scheme 1: $R_1\text{-COOH} + R_2\text{-OH} \rightarrow R_1\text{-COO-}R_2 = R_1\text{-L-}R_2$

wherein L is an ester linker -COO-, $R_1$ is a phenylbutyric acid residue and $R_2$ is the residues of the component.

Scheme 2: $R_1\text{-COOH} + R_2\text{-NH}_2 \rightarrow R_1\text{-CONH-}R_2 = R_1\text{-L-}R_2$

wherein L is an amide linker -CONH- and $R_1$ and $R_2$ have the meaning as given above.

Scheme 3:

Step 1: $R_1\text{-COOH} + \text{HO-L-CO-Prot} \rightarrow R_1\text{-COO-L-CO-Prot}$
wherein Prot is a suitable reversible protecting group.

Step 2: $R_1\text{-COO-L-CO-Prot} \rightarrow R_1\text{-COO-L-COOH}$

Step 3: $R_1\text{-COO-L-COOH} + R_2\text{-OH} \rightarrow R_1\text{-COO-L-COO}R_2$
wherein L, $R_1$ and $R_2$ have the meaning as given above.

Scheme 4:

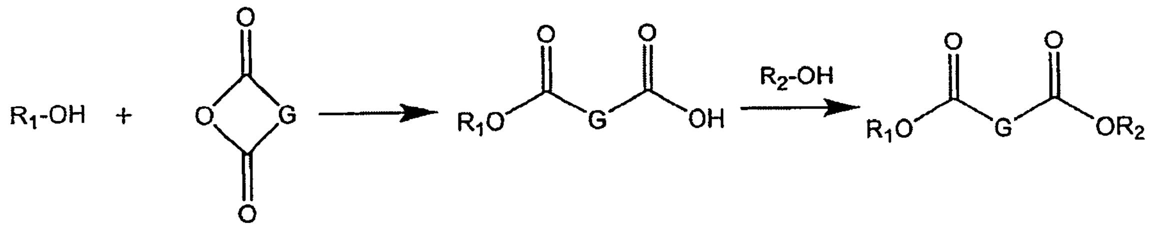


wherein $R_1$ and $R_2$ have the meanings set forth above and G is a direct bond, an $C_1$-$C_4$ alkylene, a $C_2$-$C_4$ alkenylene, a $C_2$-$C_4$ alkynylene, or a 1, 2-fused ring, and G together with the anhydride group completes a cyclic anhydride. Suitable anhydrides include succinic anhydride, glutaric anhydride, malefic anhydride, diglycolic anhydride, and phthalic anhydride.

Within the pharmaceutical composition of the invention a 4-PB codrug can be used to combine the anticancer activity of 4-PB with another pharmaceutically active compound, wherein both compounds are given in a specified stoichiometry and are transported together to the tissue of interest. Furthermore, by linking two compounds to a codrug the hydrophilicity of one or both compounds could decreased thereby improving the transport across the physiologically relevant barriers such as the skin or the blood brain barrier. For example the ester formed by 4-PB and the hydroxyl group of an anticancer compound such as curcumin has a lower hydrophilicity than the two educts and thus exhibits increased bioavailability.

**Co-crystals**

In a further aspect of the invention the pharmaceutical composition comprises a co-crystal of 4-PB.

"Co-crystal" as used herein is defined as a crystalline material comprising two or more compounds at ambient temperature (20 to 25°C, preferably 20°C), of which at least two are held together by weak interaction, wherein at least one of the compounds is a co-crystal former. Weak interaction is being defined as an interaction, which is neither ionic nor covalent and includes for example hydrogen bonds. Other modes of molecular recognition may also be present including, pi-stacking, guest-host complexation and van der Waals interactions. Of the interactions listed above, hydrogen-bonding is the dominant interaction in the formation of the co-crystal, (and a required interaction according to the present invention) whereby a non-covalent bond is formed between a hydrogen bond donor of one of the moieties and a hydrogen bond acceptor of the other. Solvates or salts of phenyl butyric acid and an organic compound that do not further comprise a co-crystal former are not co-crystals according to the present invention. The co-crystals may however, include one or more solvate molecules in the crystalline lattice. Just for the sake of clarity the distinction between crystalline salt and a co-crystal has to be stressed here. An API bound to another compound forming a salt by means of ionic interaction can be considered as one "compound" according to the invention, but it cannot be considered as two compounds by itself.

In a further embodiment the pharmaceutical composition according to the invention comprises one of the following combinations:

- a co-crystal of 4-PB and component B as co-crystal former including a respective polymorph, hydrate or solvate of the co-crystal;
- a co-crystal of 4-PB and component B as co-crystal former;
- a polymorph of a co-crystal of 4-PB and component B as co-crystal former;
- a hydrate of a co-crystal of 4-PB and component B as co-crystal former; or
- a solvate of a co-crystal of 4-PB and component B as co-crystal former.

In the scientific literature there is currently some discussion on the proper use of the word co-crystal (see for example Desiraju, CrystEngComm, 2003, 5(82), 466-467 and Dunitz, CrystEngComm, 2003, 5(91), 506). A recent article by Zaworotko (Zaworotko, Crystal Growth & Design, Vol. 7, No. 1, 2007, 4-9) gives a definition of co-crystal which is in line with the definition given above.

A "component B as co-crystal former" as used herein is defined as any compound B, which is selected from the group consisting of short chain fatty acids, NSAIDs, HDAC inhibitors not related to phenylbutyric acid, polyphenols, stilbenes, flavones and flavinoids, vitamin D derivatives, curcumin derivatives, organosulfur compounds, organoselenium compounds, polyunsaturated fatty acids and esters thereof, carotenoids, chalcone derivatives, steroids, cyclic triterpenes,

phenylpropene derivatives, monoterpenes and chemopreventive phytochemicals, being preferably a polyphenol, which is able to form a co-crystal with 4-phenylbutyric acid or its salts.

In a preferred embodiment the pharmaceutical composition comprises one of the above listed co-crystals, wherein the difference in pKA between 4-phenylbutyric acid and component B does not exceed 2.7.

In a further embodiment the pharmaceutical composition according to the invention comprises one of the following combinations:

- a co-crystal of a salt of 4-phenylbutyrate with a positively charged ion of component B and at least one organic molecule as co-crystal former including a respective polymorph, hydrate or solvate of the co-crystal;
- a co-crystal of a salt of 4-phenylbutyrate with a positively charged ion of component B and at least one organic molecule as co-crystal former;
- a polymorph of a co-crystal of a salt of 4-phenylbutyrate with a positively charged ion of component B and at least one organic molecule as co-crystal former;
- a hydrate a co-crystal of a salt of 4-phenylbutyrate with a positively charged ion of component B and at least one organic molecule as co-crystal former; or
- a solvate a co-crystal of a salt of 4-phenylbutyrate with a positively charged ion of component B and at least one organic molecule as co-crystal former.

A co-crystal can be used within the pharmaceutical composition according to the invention to improve the properties of 4-PB as compared to the 4-PB in a free form (including free acid, neutral form, hydrates, solvates, etc.), or an base salt thereof particularly with respect to: solubility, dissolution, bioavailability, stability, Cmax, Tmax, processability, longer lasting therapeutic plasma concentration, hygroscopicity, crystallization of amorphous compounds, decrease in form diversity (including polymorphism and crystal habit), change in morphology or crystal habit, etc. For example, a co-crystal form of 4-PB is particularly advantageous where the 4-PB derivative is practically insoluble or of low solublility in water. Additionally, the co-crystal properties conferred upon the 4-PB derivative are also useful because the bioavailability of the 4-PB derivative can be improved and the plasma concentration and/or serum concentration of the 4-PB derivative can be improved. This is particularly advantageous for orally-administrable formulations. Moreover, the dose response of the 4-PB can be improved, for example by increasing the maximum attainable response and/or increasing the potency of the 4-PB derivative by increasing the biological activity per dosing equivalent.

Another aspect of the present invention refers to pharmaceutical unit dosage forms, pharmaceutical compositions, pharmaceutical formulations, pharmaceutical preparations and the like comprising at least one compound of the invention, as described herein, together with one or more other pharmaceutically acceptable ingredients well known to those skilled in the art, including, but not limited to, pharmaceutically acceptable carriers, diluents, excipients, adjuvants, fillers, buffers, preservatives, anti-oxidants, lubricants, stabilisers, solubilisers, surfactants (e.g., wetting agents), masking agents, colouring agents, flavouring agents, and sweetening agents. Accordingly, the compounds and compositions of the present invention are preferably formulated in a unit dosage form, each dosage containing between 12.5 and 1500mg of 4-PB, and more preferably the amount set forth herein. The term "unit dosage form" refers to physically discrete units, such as capsules or tablets suitable as unitary dosages for human patients and other mammals, each unit containing a predetermined quantity of one or more active ingredient(s) calculated to produce the desired therapeutic effect, in association with at least one pharmaceutically acceptable carrier, diluent, excipient, or combination thereof.

For the purpose of the present invention, the expressions "a pharmaceutical unit dosage form" is synonymous and interchangeable with the expressions "unit dosage form", or "dosage form" and/or "a medicament".

The formulation may further comprise other active agents, for example, other therapeutic or prophylactic agents.

Further, another aspect of the present invention refers to a pharmaceutical composition, a pharmaceutical formulation, a pharmaceutical preparation and the like comprising 4-PB as a free acid, a prodrug, a codrug, a co-crystal or as a salt or a hydrate or solvate thereof; as an ester; or any hydrate or solvate thereof. Such pharmaceutical composition, pharmaceutical formulation or pharmaceutical preparation can either be directly employed as pharmaceutical unit dosage form or can be manufactured into such pharmaceutical unit dosage forms as described herein. Any mentioning of "formulations" or "compositions", "preparations" or the like refers to "pharmaceutical composition" and/or "a pharmaceutical formulation" or "pharmaceutical preparations" as described herein. Therefore, any teaching, disclosure, technical description and the like contained herein and reciting, referring to or specifiying pharmaceutical compositions, pharmaceutical formulations, a pharmaceutical preparations equally applies to the compositions, specifications, contents, production, use and the like of the pharmaceutical dosage unit forms of the invention.

Methods for preparing the compounds of the invention as crystalline or amorphous solids, granules, dry powders, liquid extracts (e.g. oily extracts, organic extracts, alcoholic extracts, aqueous extracts and the like) and using such preparations in the manufacture of the pharmaceutical unit dosage forms as described herein will be apparent to those skilled in the art. Liquid extracts can be used directly for preparation of liquid pharmaceutical dosage forms as described herein or dried, lyophlized and the like to yield crystalline or amorphous solids, granules, dry powders.

In general the crystalline or amorphous solids, dry powders or granules as described herein may be presented for example in a dry form in a sachet or capsules, and/or crystalline or amorphous solids, dry powders or granules maybe intended to be reconstituted to form liquid suspensions for administration to patients. Also such solids, powders or granules can be intermediate products, e.g. for subsequent compaction into tablets, pills, dragees, and for production of further solid administration forms as described herein, and/or for introduction into capsules, for example hard gelatine capsules.

Thus, the present invention provides pharmaceutical unit dosage forms, and methods of making a pharmaceutical unit dosage form comprising admixing at least one compound of the invention, as described herein, together with one or more other pharmaceutically acceptable ingredients well known to those skilled in the art, e.g., carriers, diluents, excipients, etc. If formulated as discrete units (e.g., tablets, etc.), each unit contains a predetermined amount (dosage) of the compound.

The compound of the invention may be dissolved in, suspended in, or admixed with one or more other pharmaceutically acceptable ingredients. The compound may be presented in a liposome or other microparticulate such as a polymersome which is designed to target the compound, for example, to blood components or one or more organs.

In order to deliver the 4-phenylbutyric acid across the intact skin, several approaches are known to the skilled person. One of them is to either destroy or fluidize the lipid bi-layer in the stratum corneum using hydrophobic medium such as the oil phase in an emulsion, thereby enhancing the penetration of actives.

Another approach is the use of a vesicular system such as liposomes. Liposomes are vesicles composed of phospholipid bi-layers. A phospholipid molecule has a polar "head" and two non-polar "tails". Due to its structure, the phospholipid tends to form a vesicular bi-layer, i.e. liposome, with the polar heads of phospholipids aligning toward an outer or inner aqueous phase while the non-polar tails align toward each other. Such liposomes have been used to construct carriers for the delivery of actives and can also be used for the delivery of 4-phenylbutyric acid.

As used herein, "liposome" is used to describe oligo-lamellar lipid vesicles comprising one or more natural or synthetic lipid bi-layers surrounding an internal aqueous phase.

In the context of the present invention, the term "polymersome" is defined as an artificial vesicle representing a hollow sphere that encloses a solution. Polymersomes are made using amphiphilic synthetic block copolymers to form the vesicle membrane, and have radii ranging from 50 nm to 5 $\mu$m or more.

In a preferred embodiment of the invention said microparticulate which is preferably a liposome or a polymersome comprises a prodrug or a crodrug of 4-phenylbutyric acid.

In a further preferred embodiment the prodrug or codrug is modified in a way that it adheres to the lipid layer. This adherence might be present at the inner side of the vesicle, the outer side of the vesicle or on both sides. An adherence to the inner side of the vesicle improves the loading of the vesicle during the manufacturing process and might also enable a controlled release of 4-phenylbutyric acid from the microparticulate structure.

In another preferred embodiment of the invention the prodrug or codrug is modified in a way that is incorporated into the lipid layer.

In a still further preferred adheres to the lipid layer the prodrug or codrug is construed in a way that enables the cleavage of the codrug or the prodrug in the acidic and/or enzymatic milieu of the lysosome.

The term "pharmaceutically acceptable," as used herein, pertains to compounds, ingredients, materials, compositions, dosage forms, etc., which are, within the scope of sound medical judgment, suitable for use in contact with the tissues of the subject in question (e.g., human) without excessive toxicity, irritation, allergic response, or other problem or complication, commensurate with a reasonable benefit/risk ratio. Each carrier, diluent, excipient, etc. must also be "acceptable" in the sense of being compatible with the other ingredients of the formulation. The term "pharmaceutically acceptable" can also mean approved by a regulatory agency or other generally recognized pharmacopeia for use in animals, and more particularly in humans.

Suitable carriers, diluents, excipients, etc. can be found in standard pharmaceutical texts, for example, Remington's Pharmaceutical Sciences. 18th edition, Mack Publishing Company, Easton, Pa., 1990; and Handbook of Pharmaceutical Excipients, 5th edition, 2005.

The pharmaceutical unit dosage forms may be prepared by any methods well known in the art of pharmacy. Such methods include the step of bringing into association the compound with a carrier which constitutes one or more accessory ingredients. In general, the formulations are prepared by uniformly and intimately bringing into association the compound with carriers (e.g., liquid carriers, finely divided solid carrier, etc.), and then shaping the product, if necessary.

The unit dosage form may be prepared to provide for rapid or slow release; immediate, delayed, timed, or sustained release; or a combination thereof as described herein.

A pharmaceutical unit dosage form according to the present invention may also be administered to a patient, subject or individual with the help of various delivery systems known to the person skilled in the art, e.g., via encapsulation in liposomes, microparticles, microcapsules, receptor-mediated endocytosis etc.

Methods of introduction may be topical, enteral or parenteral and may include intradermal, intramuscular,

intraperitoneal, intravenous, subcutaneous, intranasal, buccal, inhalational, epidural, and oral routes. The composition may be administered by any convenient route, for example by infusion or bolus injection, by absorption through epithelial or mucocutaneous linings (e. g., oral mucosa, rectal and intestinal mucosa, etc.) or by inhalation and may be administered together with other biologically active agents. Administration can be systemic or local.

In another embodiment the pharmaceutical unit dosage form may be delivered directly to internal organs, body cavities and the like by use of imaging devices used to guide an injecting needle directly to the site of interest. The pharmaceutical composition may also be administered to disease sites at the time of surgical intervention. In yet another embodiment, the composition can be delivered in a controlled release system.

A pharmaceutical unit dosage form according to the present invention may be desirably administered to patients through an oral route, a mucosal route (i.e. nasal, hypoglossal, vaginal, buccal, or rectal routes), a parenteral route (i.e. subcutaneous, intravenous, bolus injection, intramuscular or intraarterial routes), a topical route (i.e. eye), a transdermal route or a transcutaneous route. Examples of the single unit dose comprise, but are not particularly limited to, a liquid dose of administration suitable for oral or mucosal administration to the patients, comprising tablets; caplets; capsules such as soft elastic gelatine capsule; cachets; troches; films; lozenges; dispersants; suppositories; powders; aerosols (i.e. nasal sprays or inhalers); gels; suspensions (i.e. aqueous or non-aqueous liquid suspensions, oil-in-water emulsion, or water-in-oil liquid emulsion), solutions and elixirs; a liquid dose of administration suitable for injection to the patients; eye drops or other ophthalmological formulations for topical administration to the patients; and a sterile solid formulations (i.e. crystalline or amorphous solids) that may be reconstituted to provide the liquid dose of administration suitable for injection to the patients.

Pharmaceutical unit dosage forms may suitably be in the form of liquids, solutions (e.g., aqueous, non-aqueous), suspensions (e.g., aqueous, non-aqueous), emulsions (e.g., oil-in-water, water-in-oil), elixirs, syrups, electuaries, mouthwashes, drops, tablets (including, e.g., coated tablets), granules, powders, lozenges, pastilles, capsules (including, e.g., hard and soft gelatine capsules), cachets, pills, ampoules, boluses, suppositories, pessaries, tinctures, gels, pastes, ointments, creams, lotions, oils, foams, sprays, mists, or aerosols.

The pharmaceutical unit dosage forms may also suitably be provided as a patch, adhesive plaster, bandage, dressing, or the like which is impregnated with one or more compounds and optionally one or more other pharmaceutically acceptable ingredients, including, for example, penetration, permeation, and absorption enhancers. The pharmaceutical unit dosage forms may also suitably be provided in the form of a depot or reservoir.

Pharmaceutical unit dosage forms suitable for oral administration (e.g., by ingestion) include liquids, solutions (e.g., aqueous, non-aqueous), suspensions (e.g., aqueous, non-aqueous), emulsions (e.g., oil-in-water, water-in-oil), mouthwashes, lozenges, pastilles, as well as patches, adhesive plasters, depots, and reservoirs. elixirs, syrups, electuaries, tablets, granules, powders, capsules, cachets, pills, ampoules, boluses and films.

In a preferred embodiment the oral administration is selected from sublingual or oral transmucosal administration.

Formulations suitable for sublingual administration include tablets, lozenges, pastilles, capsules, and pills. Formulations suitable for oral transmucosal administration include liquids, solutions (e.g., aqueous, non-aqueous), suspensions (e.g., aqueous, non-aqueous), emulsions (e.g., oil-in-water, water-in-oil), and films.

Lozenges typically comprise the compound in a flavored basis, usually sucrose and acacia or tragacanth. Pastilles typically comprise the compound in an inert matrix, such as gelatin and glycerin, or sucrose and acacia. Mouthwashes typically comprise the compound in a suitable liquid carrier.

In a preferred embodiment the formulations for oral administration (e.g., by ingestion) are selected from liquids, solutions (e.g., aqueous, non-aqueous), suspensions (e.g., aqueous, non-aqueous), emulsions (e.g., oil-in-water, water-in-oil), elixirs, syrups, sprays or electuaries.

In a preferred embodiment of the invention formulations for oral administration are in the form of a spray, a gargle or mouthwash, emulsions (e.g., oil-in-water, water-in-oil), elixirs, syrups, sprays or electuaries. Such liquid pharmaceutical compositions can comprise 4-PB at a level of at least 0.0001% w/v. The composition can contain 0.0001-50%) w/v 4-PB. Preferably composition contains 0.005 to 40 w/v%, e.g. 0.05-35 w/v %, 0.5-30 w/v %, 0.7-30 w/v %, 1-25 w/v %, 1.5-30 w/v %,2-25 w/v %, 2.5-25 w/v % 4-PB.

In a preferred embodiment of the invention the liquid pharmaceutical formulations for oral administration are in the form of an aqueous solution or aqueous suspension for oral ingestion.

In a preferred embodiment the liquid pharmaceutical compositions for oral ingestion comprise 0.5 w/v %, 1 w/v %, 2 w/v %, 3 w/v %, 4 w/v %, 5 w/v %, 6 w/v % or 7 w/v % 4-PB.

Alternatively, when the formulation of this invention are presented as liquid pharmaceutical composition for oral ingestion, the amount of 4-PB included in the formulation may contain from 1.25 to 1500mg 4-PB /milliliter (expressed as the free acid), preferably from 12.5 to 1500mg 4-PB/milliliter, e.g. 12.5, 25, 50, 75, 100, 125, 150, 200, 250, 300, 400, 500, 600, 700, 800, 900, 1000, 1200, 1400, or 1500mg 4-PB/milliliter. Typically the amount of 4-PB may be in the range from 25 to 1500 mg 4-PB/milliliter, suitably from 100 to 1500mg 4-PB/milliliter, preferred from 200 to 1500mg 4-PB/milliliter, 400 to 1500mg, or 600 to 1500mg/milliliter.

Alternatively, when the formulation of this invention are presented as liquid pharmaceutical composition for oral ingestion, the amount of 4-PB included in the formulation may contain from 1.25 to 1500mg 4-PB /liter (expressed as the free acid), preferably from 12.5 to 1500mg 4-PB/ liter, e.g. 12.5, 25, 50, 75, 100, 125, 150, 200, 250, 300, 400, 500, 600, 700, 800, 900, 1000, 1200, 1400, or 1500mg 4-PB/ liter. Typically the amount of 4-PB may be in the range from 25 to 1500 mg 4-PB/ liter, suitably from 100 to 1500mg 4-PB/ liter, preferred from 200 to 1500mg 4-PB/ liter, 400 to 1500mg, or 600 to 1500mg/ liter.

Typically the composition contains a buffer. The term "buffer" refers to a pharmaceutically acceptable excipient that helps to maintain the pH of the solution within a particular range specific to the buffering system. The buffer is present for example at a concentration in the range from about 0.03%> to about 5.0% w/v, or about 0.1 % to about 2.0% w/v. Non-limiting illustrative examples of pharmaceutically acceptable buffering agents include phosphates, ascorbates, acetates, citrates, tartrates, lactates, succinates, amino acids and maleates. Particularly preferred buffers are disodium hydrogen orthophosphate, citric acid or combinations thereof.

The pH of a composition in preferred embodiments is generally from from about 6 to about 9. Typically, the pH of the liquid formulation is about 7.4. Alternatively, the pH of the liquid formulation may be selected from the following ranges: 6.5 to 8.5; 7.0 to 8.0; and 7.2 to 7.6.

The composition can further contain a thickening agent such as hydroxy ethyl cellulose, hydroxy propyl methyl cellulose, sodium carboxy methyl cellulose or hydroxy propyl cellulose.

Minor amounts of other ingredients such as pH adjusters (e.g., a base such as NaOH), emulsifiers or dispersing agents, preservatives, sweeteners, and flavourants may also be present.

A preferred liquid composition according to the present invention comprises:

(a) 0.0001 - 50% of 4-PB;

(b) up to 5% of one or more aqueous buffers;

(c) 50 - 99% Water.

The composition may further comprise up to 1% one or more flavourants, up to 0.2% sweetener and up to 0.5% preservatives.

The composition may further comprise up to 0.5% thickening agent.

Such liquid pharmaceutical compositions can comprise 4-PB at a level of at least 0.0001% w/v. The composition can contain 0.0001-50%) w/v 4-PB. Preferably composition contains 0.005 to 40 w/v%, e.g. 0.05-35 w/v %, 0.5-30 w/v %, 0.7-30 w/v %, 1-25 w/v %, 1.5-30 w/v %, 2-25 w/v %, 2.5-25 w/v % 4-PB.

In a preferred embodiment the liquid pharmaceutical compositions comprise 0.5 w/v %, 1 w/v%, 2 w/v %, 3 w/v %, 4 w/v %, 5 w/v %, 6 w/v & or 7 w/v % 4-PB.

In another preferred embodiment the formulations for oral administration are selected from tablets, pills, capsules, capsules (including, e.g., hard and soft gelatin capsules), lozenges, pastilles, cachets, films and the like.

In a more preferred embodiment the formulations for oral administration are selected from tablets or capsules (including, e.g., hard and soft gelatin capsules).

Owing to the convenience in administration, the tablet and capsule are in the most useful oral dosage unit. When preferred, the tablet may be coated using the standard aqueous or non-aqueous technologies. Such a dosage form may be prepared by any of the methods known in the field of pharmacology. In general, the pharmaceutical composition and its dosage form are prepared by uniformly and intimately mixing a liquid carrier, a finely separated solid carrier, or both of them, with an active component, and shaping a product into a desirable form, when necessary. Examples of the vehicle that may be used in the oral dosage form of the present invention include, but are not particularly limited to, a binding agent, a filling agent, a disintegrating agent, and a lubricant. The binding agent suitable for the used in the pharmaceutical composition and its dosage form includes, but is not particularly limited to, corn starch, potato starch, or other starches, natural and synthetic gums such as gelatin and acacia, sodium alginate, alginic acid, other alginates, powdered tragacanth, guar gum, cellulose and its derivatives (For example, ethyl cellulose, cellulose acetate, carboxymethyl cellulose calcium, sodium carboxymethyl cellulose), polyvinyl pyrrolidone, methyl cellulose, pre-gellated starch, hydroxypropyl methyl cellulose, (for example, Nos . 2208, 2906, 2910), microcrystalline cellulose, and mixture thereof.

A preferred solid formulation for oral administration is a tablet formulation. The term "tablet" comprises any type of tablets, pills, or dragees known in the art. Tablets can for example made by compaction of powdered, solid and/or granulated ingredients as described herein, for example including conventional excipients such as fillers, diluents, compaction aids, disintegrants, lubricants, wetting agents, flavours, colourants etc. as are known in the art. Further, tablets may be made by any conventional means, e.g., compression or moulding, optionally with one or more accessory ingredients. Compressed tablets may be prepared by compressing in a suitable machine the compound in a free-flowing form

such as a powder or granules, optionally mixed with one or more binders (e.g., povidone, gelatin, acacia, sorbitol, tragacanth, hydroxypropylmethyl cellulose); fillers or diluents (e.g., lactose, microcrystalline cellulose, calcium hydrogen phosphate); lubricants (e.g., magnesium stearate, talc, silica); disintegrants (e.g., sodium starch glycolate, cross-linked povidone, cross-linked sodium carboxymethyl cellulose); surface-active or dispersing or wetting agents (e.g., sodium lauryl sulfate); preservatives (e.g., methyl p-hydroxybenzoate, propyl p-hydroxybenzoate, sorbic acid); flavours, flavour enhancing agents, and sweeteners. Moulded tablets may be made by moulding in a suitable machine a mixture of the powdered compound moistened with an inert liquid diluent. The tablets may optionally be coated or scored and may be formulated so as to provide slow or controlled release of the compound therein using, for example, hydroxypropylmethyl cellulose in varying proportions to provide the desired release profile. Tablets may optionally be provided with a coating, for example, to affect release, for example an enteric coating, to provide release in parts of the gut other than the stomach.

A disintegrating agent is used in the tablets of the present invention to disintegrate the composition when the tablet is exposed to an aqueous environment. The tablet containing a very large amount of the disintegrating agent may be disintegrated during a storage period, but the tablet containing a very small amount of the disintegrating agent may not be disintegrated at a desirable rate under a preferred condition. Therefore, a suitable amount of the disintegrating agent to control the release of active components should be used to form a solid oral dosage form of the present invention. The amount of the used disintegrating agent is varied according to the type of the formulation, and may be easily determined by those skilled in the art to which the present invention belongs. The typical pharmaceutical composition comprises approximately 0.5 to 15 % by weight of a disintegrating agent, preferably approximately 1 to 5 % by weight of a disintegrating agent.

Pharmaceutical unit dosage forms in the form of tablets can be coformulated with a pharmaceutically acceptable methacrylic copolymer. Suitable generic classes and specific examples of pharmaceutically acceptable methacrylic acid copolymer are known polymers which are copolymers of acrylic and methacrylic esters with a low content of quaternary ammonium groups, for example having a molar ratio of such ammonium groups : remaining (meth)acrylic esters of 1 : > 10, for example around 1 : 20 or 1 : 40, and with a mean molecular weight of around 150000.

Further suitable generic classes and specific examples of pharmaceutically acceptable methacrylic acid copolymer are known polymers which are copolymers, anionic in character, based on methacrylic acid and methyl methacrylate, for example having a ratio of free carboxy 1 groups : methyl-esterified carboxy 1 groups of 1:< 3, e.g around 1:1 or 1:2, and with a mean molecular weight of 135000. Such polymers are sold under the trade name Eudragit ™, such as the Eudragit L series e.g. Eudragit L 12.5 ™, Eudragit L 12.5P ™, Eudragit L100 ™, Eudragit L 100-55 ™, Eudragit L-30 D ™, Eudragit L-30 D-55 ™, the Eudragit S ™ series e.g. Eudragit S 12.5, Eudragit S 12.5P ™, Eudragit S100 ™, the Eudragit NE™ series e.g Eudragit NE 30D ™, the Eudragit RL ™ series, e.g. Eudragit RL 12.5 ™, Eudragit RL 100 ™, Eudragit RL PO ™, Eudragit RL 30D ™, and the Eudragit RS ™ series e.g. Eudragit RS 12.5 ™, Eudragit RS 100 ™, Eudragit RS PO™, and Eudragit RS 30D™ Some of these polymers are enteric polymers (the term "enteric polymer" is a term of the art referring to a polymer which is preferentially soluble in the less acid environment of the intestine relative to the more acid environment of the stomach), for example having a solubility in aqueous media at pH 5.5 and above.

Other suitable pharmaceutically acceptable polymers include for example methyl acrylate-methacrylic acid copolymer, methacrylate-methacrylic acid-octyl acrylate copolymer, etc. These may be used either alone or in combination, or together with other polymers than those mentioned above. Such tablets may also include an effervescent couple, and/or a chewable base. Such tablets may be made by processes well known in the art, for example blending of powdered constituents, granulation e.g. by slugging or roller compaction, then compaction into a tablet form in a conventional tablet press.

In one embodiment the tablet containing the compound of the invention as described herein is an immediate release tablet. In classical terms, an immediate release formulation releases at least 80% of its active pharmaceutical ingredient within 30 minutes. With reference to the present invention, the definition of an immediate release formulation will be broadened further to include a formulation which releases more than about 80% of its active pharmaceutical ingredient within 60 minutes in a standard USP Paddle Method dissolution test at 50 rpm in 500 ml media having a pH of between 1.2 and 6.8 at 37°C.

In another embodiment the tablet containing the compound of the invention as described herein, is a controlled release tablet. "Controlled release" formulations, as referred to herein, will then encompass any formulations which release no more than about 80% of their active pharmaceutical ingredients within 60 minutes under the same conditions. The controlled release tablet of this invention exhibits a dissolution rate *in vitro,* when measured by USP Paddle Method at 50 rpm in 500 ml media having a pH between 1.2 and 6.8 at 37°C, of about 15% to about 50% by weight 4-PB released after 1 hour, about 45% to about 80% by weight 4-PB released after 4 hours, and at least about 80% by weight 4-PB released after 10 hours.

The release technology is selected from the group consisting of sustained-release (SR), sustained-action (SA), extended-release (ER, XR, or XL), time-release or timed-release, modified release (MR) or continuous release (CR) tablet.

The person skilled in the art is aware of the different technologies used to formulate time-release drugs, e.g. so that the active ingredient is embedded in a matrix of insoluble substance(s) (acrylics, chitin etc.), such that the dissolving drug must find its way out through the holes in the matrix. Also 4-PB enclosed in polymer-based tablets with a laser-drilled hole on one side and a porous membrane on the other side is envisioned. In SR formulations, the compounds of the invention can be made to dissolve into the matrix, and the matrix physically swells to form a gel, allowing the drug to exit through the gel's outer surface. Also micro-encapsulation of the present compounds is envisioned, providing as a more complete technology to produce complex dissolution profiles. Through coating an active pharmaceutical ingredient around an inert core, and layering it with insoluble substances to form a microsphere more consistent and replicable dissolution rates can be obtained.

The preferred composition for achieving and maintaining the proper blood plasma levels is a controlled-release matrix. In this embodiment, the 4-PB is dispersed in a controlled release delivery system that comprises a hydrophilic material (gelling agent) which upon exposure to gastrointestinal fluid forms a gel matrix that releases 4-PB at a controlled rate. Such hydrophilic materials include gums, cellulose ethers, acrylic resins, and protein-derived materials. Suitable cellulose ethers include hydroxyalkyl celluloses and carboxyalkyl celluloses, especially hydroxyethyl cellulose (HEC), hydroxypropyl cellulose (HPC), HPMC, and carboxy methylcellulose (CMC). Suitable acrylic resins include polymers and copolymers of acrylic acid, methacrylic acid, methyl acrylate and methyl methacrylate. Suitable gums include heteropolysaccharide and homopolysaccharide gums, e.g., xanthan, tragacanth, acacia, karaya, alginates, agar, guar, hydroxypropyl guar, carrageenan, and locust bean gums.

Preferably, the controlled release tablet of the present invention is formed from (I) a hydrophilic material comprising (a) a heteropolysaccharide; or (b) a heteropolysaccharide and a cross-linking agent capable of cross-linking said heteropolysaccharide; or (c) a mixture of (a), (b) and a polysaccharide gum; and (II) an inert pharmaceutical filler comprising up to about 80% by weight of the tablet; and (III) 4-PB.

The term "heteropolysaccharide" as used herein is defined as a water-soluble polysaccharide containing two or more kinds of sugar units, the heteropolysaccharide having a branched or helical configuration, and having excellent water-wicking properties and immense thickening properties.

A preferred heteropolysaccharide is xanthan gum, which is a high molecular weight $>10^6$) heteropolysaccharide. Other preferred heteropolysaccharides include derivatives of xanthan gum, such as deacylated xanthan gum, the carboxymethyl ether, and the propylene glycol ester.

The cross linking agents used in the controlled release embodiment of the present invention which are capable of cross-linking with the heteropolysaccharide include homopolysaccharide gums such as the galactomannans, i.e., polysaccharides which are composed solely of mannose and galactose. Galactomannans which have higher proportions of unsubstituted mannose regions have been found to achieve more interaction with the heteropolysaccharide. Locust bean gum, which has a higher ratio of mannose to the galactose, is especially preferred as compared to other galactomannans such as guar and hydroxypropyl guar.

Preferably, the ratio of heteropolysaccharide to homopolysaccharide is in the range of about 1:9 to about 9:1, preferably about 1:3 to about 3:1. Most preferably, the ratio of xanthan gum to polysaccharide material (i.e., locust bean gum, etc.) is preferably about 1:1.

In addition to the hydrophilic material, the controlled release delivery system can also contain an inert pharmaceutical diluent such as a monosaccharide, a disaccharide, a polyhydric alcohol and mixtures thereof. The ratio of diluent to hydrophilic matrix-forming material is generally in the range of about 1:3 to about 3:1.

The controlled release properties of the controlled release embodiment of the present invention may be optimized when the ratio of heteropolysaccharide gum to homopolysaccharide material is about 1:1, although heteropolysaccharide gum in an amount of from about 20 to about 80% or more by weight of the heterodisperse polysaccharide material provides an acceptable slow release product. The combination of any homopolysaccharide gums known to produce a synergistic effect when exposed to aqueous solutions may be used in accordance with the present invention. It is also possible that the type of synergism which is present with regard to the gum combination of the present invention could also occur between two homogeneous or two heteropolysaccharides. Other acceptable gelling agents which may be used in the present invention include those gelling agents well-known in the art. Examples include vegetable gums such as alginates, carrageenan, pectin, guar gum, xanthan gum, modified starch, hydroxypropylmethylcellulose, methylcellulose, and other cellulosic materials such as sodium carboxymethylcellulose and hydroxypropyl cellulose. This list is not meant to be exclusive. The chemistry of certain of the ingredients comprising the excipients of the present invention such as xanthan gum is such that the excipients are considered to be self-buffering agents which are substantially insensitive to the solubility of the medicament and likewise insensitive to the pH changes along the length of the gastrointestinal tract.

The inert filler of the sustained release excipient preferably comprises a pharmaceutically acceptable saccharide, including a monosaccharide, a disaccharide, or a polyhydric alcohol, and/or mixtures of any of the foregoing. Examples of suitable inert pharmaceutical fillers include sucrose, dextrose, lactose, microcrystalline cellulose, fructose, xylitol, sorbitol, mixtures thereof and the like. However, it is preferred that a soluble pharmaceutical filler such as lactose,

dextrose, sucrose, or mixtures thereof be used.

The cationic cross-linking agent which is optionally used in conjunction with the controlled release embodiment of the present invention may be monovalent or multivalent metal cations. The preferred salts are the inorganic salts, including various alkali metal and/or alkaline earth metal sulfates, chlorides, borates, bromides, citrates, acetates, lactates, etc. Specific examples of suitable cationic cross-linking agents include calcium sulfate, sodium chloride, potassium sulfate, sodium carbonate, lithium chloride, tripotassium phosphate, sodium borate, potassium bromide, potassium fluoride, sodium bicarbonate, calcium chloride, magnesium chloride, sodium citrate, sodium acetate, calcium lactate, magnesium sulfate and sodium fluoride. Multivalent metal cations may also be utilized. However, the preferred cationic cross-linking agents are bivalent. Particularly preferred salts are calcium sulfate and sodium chloride. The cationic cross-linking agents of the present invention are added in an amount effective to obtain a desirable increased gel strength due to the cross-linking of the gelling agent (e.g., the heteropolysaccharide and homopolysaccharide gums). In preferred embodiments, the cationic cross-linking agent is included in the sustained release excipient of the present invention in an amount from about 1 to about 20% by weight of the sustained release excipient, and in an amount about 0.5% to about 16% by weight of the final dosage form.

In the controlled release embodiments of the present invention, the sustained release excipient comprises from about 10 to about 99% by weight of a gelling agent comprising a heteropolysaccharide gum and a homopolysaccharide gum, from about 1 to about 20% by weight of a cationic crosslinking agent, and from about 0 to about 89% by weight of an inert pharmaceutical diluent. In other embodiments, the sustained release excipient comprises from about 10 to about 75% gelling agent, from about 2 to about 15% cationic crosslinking agent, and from about 30 to about 75% inert diluent. In yet other embodiments, the sustained release excipient comprises from about 30 to about 75% gelling agent, from about 5 to about 10% cationic cross-linking agent, and from about 15 to about 65% inert diluent.

The sustained release excipient used in this embodiment of the present invention (with or without the optional cationic cross-linking agent) may be further modified by incorporation of a hydrophobic material which slows the hydration of the gums without disrupting the hydrophilic matrix. This is accomplished in preferred embodiments of the present invention by granulating the sustained release excipient with the solution or dispersion of a hydrophobic material prior to the incorporation of the medicament. The hydrophobic polymer may be selected from an alkylcellulose such as ethylcellulose, other hydrophobic cellulosic materials, polymers or copolymers derived from acrylic or methacrylic acid esters, copolymers of acrylic and methacrylic acid esters, zein, waxes, shellac, hydrogenated vegetable oils, and any other pharmaceutically acceptable hydrophobic material known to those skilled in the art. The amount of hydrophobic material incorporated into the sustained release excipient is that which is effective to slow the hydration of the gums without disrupting the hydrophilic matrix formed upon exposure to an environmental fluid. In certain preferred embodiments of the present invention, the hydrophobic material is included in the sustained release excipient in an amount from about 1 to about 20% by weight. The solvent for the hydrophobic material may be an aqueous or organic solvent, or mixtures thereof.

Examples of commercially available alkylcelluloses are Aquacoat coating (aqueous dispersion of ethylcellulose available from FMC of Philadelphia, PA) and Surelease coating (aqueous dispersion of ethylcellulose available from Colorcon of West Point, PA). Examples of commercially available acrylic polymers suitable for use as the hydrophobic material include Eudragit RS and RL polymers (copolymers of acrylic and methacrylic acid esters having a low content (e.g., 1:20 or 1:40) of quaternary ammonium compounds available from Rohm America of Piscataway, NJ.).

The controlled release matrix useful in the present invention may also contain a cationic cross-linking agent such as calcium sulfate in an amount sufficient to crosslink the gelling agent and increase the gel strength, and an inert hydrophobic material such as ethyl cellulose in an amount sufficient to slow the hydration of the hydrophilic material without disrupting it. Preferably, the controlled release delivery system is prepared as a pre-manufactured granulation.

In one specific embodiment of the controlled release matrix form of the invention, the 4-PB is dispersed in a controlled release delivery system that comprises a hydrophilic material which, upon exposure to gastrointestinal fluid, forms a gel matrix that releases 4-PB at a controlled rate. The rate of release of 4-PB from the matrix depends on the drug's partition coefficient between components of the matrix and the aqueous phase within the gastrointestinal tract. In a preferred form of this embodiment, the hydrophilic material of the controlled release delivery system comprises a mixture of a heteropolysaccharide gum and an agent capable of cross-linking the heteropolysaccharide in the presence of gastrointestinal fluid. The controlled release delivery system may also comprise a water-soluble pharmaceutical diluent mixed with the hydrophilic material. Preferably, the cross-linking agent is a homopolysaccharide gum and the inert pharmaceutical diluent is a monosaccharide, a disaccharide, or a polyhydric alcohol, or a mixture thereof.

In a specific preferred embodiment, the weight ratio of heteropolysaccharide to homopolysaccharide is in the range of about 1:3 to about 3:1, the weight ratio of heteropolysaccharide to diluent is in the range of about 1:8 to about 8:1, and the weight ratio of heteropolysaccharide to 4-PB is in the range of about 10:1 to about 1:10. A preferred heteropolysaccharide is xanthan gum and a preferred homopolysaccharide is locust bean gum. The dosage form may also comprise a cationic cross-linking agent and a hydrophobic polymer.

The invention also includes a method of making an 4-PB controlled release tablet of the invention which comprises mixing particles of 4-PB or a pharmaceutically acceptable salt of 4-PB with granules comprising the controlled release delivery system, preferably followed by directly compressing the mixture to form tablets.

The invention also explicitly relates to any other oral dosage forms for controlled release comprising 4-PB, comprising capsules, pills, dragees, and the like and wherein the release form is selected from the group consisting of sustained-release (SR), sustained-action (SA), extended-release (ER, XR, or XL), time-release or timed-release, modified release (MR) or continuous release (CR) dosage forms.

The 4-PB controlled release oral solid dosage form of this invention can be made using any of several different techniques for producing controlled release oral solid dosage forms.

In one embodiment, a core comprising 4-PB or 4-PB salt is coated with a controlled release film which comprises a water insoluble material and which upon exposure to gastrointestinal fluid releases 4-PB from the core at a controlled rate.

In a second embodiment, the 4-PB or 4-PB salt is dispersed in a controlled release delivery system that comprises a hydrophilic material which upon exposure to gastrointestinal fluid forms a gel matrix that releases 4-PB at a controlled rate.

In a third embodiment is a combination of the first two: a controlled release matrix coated with a controlled release film.

In any of these embodiments, the dosage form can be a tablet, a plurality of granules, or other suitable form, and can contain lubricants, colorants, diluents, and other conventional ingredients.

In a preferred embodiment the solid oral dosage form is a microcapsule, a hard gelatine capsule or soft gelatin capsule.

In one embodiment the capsule comprising 4-PB is an immediate release capsule. In classical terms, an immediate release formulation releases at least 80% of its active pharmaceutical ingredient within 30 minutes. With reference to the present invention, the definition of an immediate release capsule will be broadened further to include a formulation which releases more than about 80% of its active pharmaceutical ingredient within 60 minutes in a standard USP Paddle Method dissolution test at 50 rpm in 500 ml media having a pH of between 1.2 and 6.8 at 37°C.

In another embodiment the capsule containing the compound of the invention as described herein, is a controlled release capsule comprising a pharmaceutical formulation of 4-PB. "Controlled release" capsules, as referred to herein, will then encompass any formulations which release no more than about 80% of their active pharmaceutical ingredients within 60 minutes under the same conditions.The controlled release dosage form of this invention exhibits a dissolution rate *in vitro,* when measured by USP Paddle Method at 50 rpm in 500 ml media having a pH between 1.2 and 6.8 at 37°C, of about 15% to about 50% by weight 4-PB released after 1 hour, about 45% to about 80% by weight 4-PB released after 4 hours, and at least about 80% by weight 4-PB released after 10 hours.

The release technology is selected from the group consisting of sustained-release (SR), sustained-action (SA), extended-release (ER, XR, or XL), time-release or timed-release, modified release (MR) or continuous release (CR) capsule.

In a particularly preferred embodiment the capsule is a controlled release capsule as described herein, the capsule having permeable or semi-permeable walls which have the ability to release 4-PB at a predetermined rate.

In an embodiment of the invention the controlled release capsules can contain 4-PB in crystalline or amorphous solids, granules, dry powders and the like as described herein.

In an embodiment of the invention the capsules can contain 4-PB as a liquid extract or the like, or a liquid formulation of 4-PB reconstituted from solids, granules, dry powders to form liquid suspensions as described herein.

The release of 4-PB from such controlled release capsules is an effect resembling osmosis, while the release of the volatile liquid is an effect produced by partial pressure differences across the capsule walls. This invention provides capsules which have permeable or semi-permeable walls, i.e. walls having microscopic passages or interconnecting pores providing a release route which permits an osmosis-like effect to occur at a controllable or predetermined rate. The observed effect resembles osmosis in that the amount of liquid contained within the capsule walls does not appear to diminish significantly, but any solute dissolved in the encapsulated liquid passes out of the capsules when the environment (e.g. stomach fluid, intestinal fluid and the like) containing a lower concentration of solute exists outside the capsules.

It has been found that a microporous capsule wall can be obtained when certain critical phase relationships are observed during the manufacture of the capsules. The capsule shell- or wall-forming material should comprise at least a first material which melts at elevated or moderately elevated temperatures and is capable of forming a single phase when admixed with a second material (which also melts at elevated or moderately elevated temperatures). A small amount of a high-boiling solvent or plasticizing material can be used, if desired, to facilitate this single phase formation step. After the first and second materials have been blended, heated, and formed into a homogeneous molten phase, the wall forming composition is brought into contact with a fill material (preferably an aqueous liquid) by means of biliquid column technique. Contact with the liquid fill rapidly cools the homogeneous wall-forming phase, and as this molten phase approaches a solidified state and becomes an incipient capsule wall or shell, one of the materials present

in this incipient wall begins to separate out as a discontinuous, solid phase dispersed throughout a substantially continuous wall matrix. The resulting dispersed particles are normally considerably smaller than the thickness of the capsule wall which ultimately results. The dispersed particles preferably contract, upon further cooling, at a rate which is faster than the contraction rate of the continuous matrix, but any disparity between the matrix and dispersed phase with regard to their respective rates or degrees of contraction can assist in the formation of cracks or pores in the capsule walls. Since the dispersed phase is present in a significant quantity, e.g. at least 5 parts by weight of the total composition, the resulting capsule is characterized by microporous walls having a porosity (determined by mercury porosimetry) of at least about 3 volume percent. The "microporosity" (this term being used herein to include sub-microscopic porosity) imparts permeability or semi-permeability to the capsule walls, and effects similar to osmosis are observed when a concentration gradient across the capsule wall is present. However, these osmosis-like effects are not necessarily limited to the diffusion of solutes; emulsoids and dispersoids also appear to pass through the capsule walls, indicating a variety of transport mechanisms may be operating. It will be understood, in any event, that this invention is not bound by any theory.

The capsules of the present invention are on the order of about 100 to about 10,000 microns, preferably about 1,000 to about 3,000 microns, in diameter. Capsules of this size are readily packaged, stored, and shipped, while larger capsules are weak, fragile, and subject to breakage during packaging, handling, or shipping. Capsules smaller than 100 microns in diameter have a large shell or wall volume in comparison to liquid fill volume and are excessively expensive for most uses. The capsule shell wall thickness varies from about 0.85 to about 10 microns for a 100 micron capsule diameter, from about 35 to about 400 microns for a 4,000 micron capsule diameter, and from about 85 to about 1,000 microns for a 10,000 micron capsule diameter. These capsule shells provide a volume ratio of contained fill to shell sufficiently high for efficient economical use.

The preferred capsules comprise an aqueous fill and a microporous (including sub-microscopic porosity) capsule wall wherein the capsule wall comprises three or more phases, i.e.: a solid, crystalline olefinic polymer phase, an amorphous phase (e.g. a hydrocarbon resin), and a second crystalline or semi-crystalline phase, e.g. a natural, paraffinic or microcrystalline petroleum wax. In this preferred composition, the wax ordinarily separates out first upon cooling, thus forming a crystalline or semi-crystalline phase dispersed throughout a continuous matrix of the other phases.

Microporosity results upon further cooling, due apparently to one or more phenomena, including differences in the rate or degree of contraction between two or more phases and shear forces created by the disparity in structure between the different crystal types or between the polyolefin/amorphous resin and wax phases. An important factor contributing to the formation of microporous capsule walls involves differences in rates or extent of contraction of materials used to form the capsule wall or shell.

In the preferred embodiments of this invention, the capsule wall-forming material is a blend of crystalline olefinic polymer; an amorphous, thermoplastic organic resin; and a wax. If X represents the weight fraction of polyolefin, Y represents the weight fraction of amorphous resin, and Z represents the weight fraction of wax; and $V_x$ represents the fractional volume contraction (defined previously) of polyolefin, Vy represents the fractional volume contraction of amorphous resin, and $V_z$ represents the fractional volume contraction of the wax, the expression:

$$\frac{XVx + XVy + XVz}{Vx + Vy}$$

will be greater than 1 when at least some wax is included in the composition, as is preferred. The expression, of course, approaches 1.0 as the wax concentration approaches zero. Zero wax concentrations result in very low mercury porosimeter readings at the low end of measurable pore size, particularly for the walls of perfect capsules. Grosser pores are sometimes evident in these wax-free capsule walls, but this is a much less preferred type of pore structure. (The solute diffusion rate can be conveniently measured by placing a given weight of capsules in a container with a given volume of water and monitoring the increasing concentration or weight gain of solute in the water, as will be described in detail subsequently.

It will be apparent that the above-noted expression compares the contraction of the total system to the contraction of the polyolefin/amorphous portion of the system. For convenience, this mathematical expression will be hereinafter referred to as the volume contraction ratio, or "VCR". Although capsules with a measurable release rate can be obtained when the VCR is 1.0 significant improvements in reproducibility and controllability of the capsule wall porosity and solute release rate are obtained when the VCR is greater than 1, preferably greater than 1.2. Apparently, a slight microporosity-creating phase separation effect can occur even when there is only one crystalline and one amorphous phase in the wall-forming system. However, at least three phases provide additional, readily controllable microporosity-creating effects. Crystal structure studies indicate that as the three-component melt cools from the temperature of capsule manufacture, the major (preferably continuous) polyolefin/amorphous resin phase comes out of solution last, leaving dispersed

zones of a crystalline or semi-crystalline wax. Further loss of heat from the capsule wall or shell causes the polyolefin/amorphous portion and the wax portion to continue to contract. The aforementioned volume contraction ratio being greater than 1, microscopic (probably including some sub-microscopic) flaws, cracks, pores, channels, or the like are created in the capsule wall with no significant detrimental side effects, e.g. no noticeable increase in weak, imperfect, excessively frangible, or leaky capsules. At least some of the microscopic cracks, pores, etc. will completely traverse the capsule wall, either by a straightforward passage or a tortuous route, and capsules with microporous walls are obtained. The existence of microporosity is confirmed by porosimeter studies, using an Aminco-Winslow mercury porosimeter, Model S-7107 or S-7108.

The presence of solute in the capsule walls can be demonstrated by analysis, using a scanning electron microscope having an x-ray detecting feature. One set of analyzed capsules originally contained an aqueous copper sulfate fill solution (25% by weight cupric sulfate-pentahydrate) and were used in a solute release rate test (3 days of leaching into water). The capsules were dried and cut in half. Copper was analyzed for, and detected at, four points in the capsule wall cross-section, ranging from the interior of the wall to its exterior, indicating an actual pass-through of cupric ion from the interior of the capsule toward the exterior.

Based on all the available evidence, one can envision a phase comprising amorphous resin molecules intimately associated with large polyolefin crystals, this polyolefin/amorphous resin phase surrounding a paraffin wax crystal. As the two crystal-containing phases contract, a shearing action takes place between the two crystal types. When the second crystalline phase (i.e. the wax) is omitted, differential rates of contraction are still possible, but the aforementioned shearing action will probably be lost. Crystalline materials such as polyolefin exhibit a greater percent volume contraction than the amorphous materials. In the context of this invention, the percent volume contraction of the polyolefin will ordinarily be at least 1.2 times greater, and preferably about 2 to 3 times greater than the amorphous resin contraction.

Thus, the capsule membrane (wall or shell) material of this invention, which provides the controlled release feature, is a multi-component blend, preferably a blend of resins and wax. The resin/wax mixture can be prepared so as to tailor the release rate of the encapsulated active ingredient to its desired application. A wide variety of adjustments in the release rate can be made by varying the composition of the capsule walls, both as to the physical properties of the selected ingredient and its weight fraction in the shell-forming melt. Since primarily physical rather than chemical phenomena are involved in the practice of this invention (in fact, chemical interaction between components is not preferred), capsule release rates can be controlled or adjusted by reference to easily calculated or observed criteria. Anomalous results are likely to be observed, of course, when the capsule wall-forming system is: at a eutectic point, so rich in wax as to produce the wax phase inversion described previously, capable of forming a plurality of dispersed phases, or incapable of forming a continuous matrix for a dispersed phase. These anomalous results may entail a decrease in capsule wall microporosity or some other poorly correlated or undesirable effect, but not necessarily a loss of the solute release feature of this invention.

In a preferred embodiment the crystalline polyolefins found useful in the capsule shells of this invention are those which have a specific gravity of about 0.90 to about 0.98, preferably about 0.91 to about 0.95, as determined by the density gradient technique (ASTM Test D 1505-63E). These polyolefins have been found to have molecular weights of about 1,000 to about 4,000, preferably about 1,500 to about 3,500, and exhibit an average viscosity of less than 500 cps at 140° C. (Brookfield viscometer, Model LVT). Polyolefins of higher specific gravity do not appear to be capable of producing as high a porosity in the capsule wall, while polyolefins of lower specific gravity are not sufficiently self-supporting to provide strong capsules. The preferred polyolefins are highly crystalline. The term "crystalline", as used herein, characterizes those olefin polymers which have a definite visible crystal structure as observed through a petrographic microscope. This crystallinity is at least partly responsible for the high fractional volume contraction ($V_x$) of these polymers, e.g. about 0.15 to about 0.25.

The term "polyolefin" or "olefin polymer" is intended to include homopolymers and those copolymers (including terpolymers, etc.) which have polyolefin character, particularly those which have some crystallinity. Generally speaking, copolymerization interferes with crystal structure; however, if at least about 50 weight percent of the polymer comprises repeating alkylene units (e.g., units derived from ethylene, propylene, 1-butene, or the like) where the polymer consists of at least 75 mole percent of such repeating alkylene units, some crystallinity will be retained, permitting the use of up to 25 mole percent of co-monomers such as vinyl acetate, acrylic acid, acrylate ester, vinyl chloride, etc.. Higher mole percentages of the monomers vinyl fluoride, vinyl alcohol, and carbon monoxide, are permissible in polyethylene copolymers.

The following is a list of typical commercially available ethylene polymers useful in the invention and their fractional volume contractions ($V_x$). All of these polymers are manufactured by Allied Chemical Plastics Division and have "AC" commercial grade numbers. Polymer grades AC 617, AC 7, and AC 8 are non-emulsifiable polyethylenes. Grades AC 656, AC 629, and AC 655 are emulsifiable polyethylenes. Grades AC 400, AC 401, and AC 405 are ethylene-vinyl acetate copolymers.

| Grade | Melt. Pt. (°C) | Density (g/cc) | Vx |
|---|---|---|---|
| AC617 | 102 | 0.91 | 0.180 |
| AC 7 | 107 | 0.92 | 0.227 |
| AC 8 | 116 | 0.93 | 0.223 |
| AC 656 | 96 | 0.92 | 0.193 |
| AC 629 | 101 | 0.93 | 0.180 |
| AC 655 | 104 | 0.93 | 0.197 |
| AC 400 | 95 | 0.92 | 0.165 |
| AC 401 | - | - | 0.183 |
| AC 405 | - | - | 0.180 |

The amorphous organic resins utilized in the capsule shells of this invention can be one or more of a broad group of materials which are compatible at elevated temperatures at the desired ratio with the polyolefin. By "elevated temperatures" is meant the temperature of capsule manufacture which normally is at least above the melting point of the highest-melting component of the capsule wall-forming composition, preferably at least 100° C. above this highest melting point. The melting points or melting ranges of the preferred amorphous thermoplastic resins are normally in the range of about 50° to about 150° C., preferably between about 85 to about 115° C. It is preferred that the amorphous resins be selected to have a melting point or melting range near the melting point of the polyolefin. The preferred resins belong to a class of materials referred to in industry by the term "hydrocarbon resins". Hydrocarbon resins are defined by the Kirk-Othmer Encyclopedia of Chemical Technology, Second Edition, Volume 11, John Wiley & Sons, New York, New York, 1966, page 242 et seq., as the readily thermoplastic polymers of low molecular weight derived from coal-tar fractions, from deeply cracked petroleum distillates, and from turpentine. These hydrocarbon resins (which are not hydrocarbon in the strictest sense of the term, since they may contain minor amounts of oxygen or other elements occurring in these natural materials) generally have a molecular weight of about 800 to about 4,000, preferably about 1,000 to about 2,000. Typical hydrocarbon resins (as defined by Kirk-Othmer) useful in the practice of this invention include coumarone-indene resins, indene resins, natural and synthetic terpene-based resins, alkyl-aromatic thermoplastic hydrocarbon resins, vinyl arene resins (based on polymers and copolymers of vinyl toluene, styrene, vinyl naphthalene, etc.), wood rosins, asphaltic resins, and other resins described by Kirk-Othmer. Natural and synthetic polyterpene is particularly useful and is commercially available as "Wing-Tack 95" (Goodyear Tire and Rubber Co.), and the various "Piccolyte" resins available from Pennsylvania Industrial Chemical Corporation. From the standpoint of obtaining high compatibility with polyolefins and high porosity in capsule walls, the "Piccopale" resins (Pennsylvania Industrial Chemical Corporation) have been found to be particularly suitable. The Piccopale resins are produced by high temperature cracking of petroleum, which produces a mixture of monomers averaging about 90 in molecular weight, including dienes and reactive olefins. Polymerization of this olefinic material produces resins with substantially straight-chain hydrocarbon backbones and some cyclic content, but little or no aromatic content.

The amount of hydrocarbon resin and crystalline polyolefins in the capsule wall-forming material are variable but to some extent interrelated. To avoid an excessive contraction rate in the polyolefin/amorphous resin phase, the polyolefin content should not exceed about 95% by weight of the capsule shell or wall composition. The amount of hydrocarbon resin is preferably at least about 5% by weight of the total composition for the same reason. It is permissible to lower the polyolefin content to about 15 or 20 % by weight, provided that the previously described phase relationships can be properly maintained. These phase relations are most easily obtained when the ratio of polyolefin to amorphous resin is at least 1:1. The amount of wax which can be added is somewhat limited, as will be explained subsequently. It is therefore generally true that drastically decreasing the polyolefin content may require increasing the hydrocarbon resin content and losing some of the diversity in crystal structure between at least two predominantly crystalline phases.

These hydrocarbon resins used in this invention exhibit a fractional volume contraction (Vy) which is less than the $V_x$ and is preferably in the 0.10-0.15 range.

The preferred amount of amorphous hydrocarbon resin ranges from about 5 to about 45% by weight of the total capsule wall-forming composition.

Other commercially available amorphous thermoplastic hydrocarbon resins include the following materials available from Pennsylvania Industrial Chemical Corporation: "Piccoumaron" resins (polyindine type), "Piccovar" resins (alkyl-aromatic type), "Piccotex" resins (vinyl toluene copolymers), and "Piccolastic" resins (low molecular weight polystyrene type). The fractional volume contraction (Vy) of the aforementioned Wing-Tack 95 is 0.102.

The fractional volume contractions (Vy) of the aforementioned Piccopale and Piccolyte resins vary slightly depending on the melting points or ranges of the resins. The density of all the common available grades (Piccopales 70 SF, 85 SF, and 100 SF melting at about 70°, 85°, and 100° C, respectively, and Piccolytes S-40, S-85, S-100, and S-135, melting at about 40°, 85°, 100°, and 135° C, respectively) is in the range of 0.96-0.98 gram per cubic cm (g/cc) and the Vy values are as follows:

| "Piccopale" | "Piccolyte" | $V_y$ |
|---|---|---|
| 70 SF | - | 0.1133 |
| 85 SF | - | 0.1200 |
| 100 SF | - | 0.1333 |
| - | S-40 | About 0.13 |
| - | S-85 | 0.120 |
| - | S-100 | 0.1333 |
| - | S-135 | About 0.12 |

The third component of the preferred composition comprises a wax or mixture of waxes, including the natural and/or petroleum and/or synthetic waxes. Again, the wax should be compatible with the other two components at the capsule manufacturing temperature, which temperature will normally be higher than the melting point of the highest-melting component of the mixture used to form the capsule wall. Crystalline paraffinic or other highly crystalline waxes are preferred, the microcrystalline petroleum waxes, the animal and vegetable waxes, the synthetic ester or amide-type waxes, etc. being less preferred. The weight fraction of wax in the capsule wall-forming composition preferably ranges from about 2 to about 25 weight percent. Generally speaking, increasing amounts of wax produce increasing porosity, up to the phase inversion condition described previously. Accordingly, the upper limit of wax concentration is not numerically fixed, but may vary to some degree with the system selected. Stated another way, the quantity of wax should be selected such that the volume contraction ratio (VCR) is at least about 1.2, but preferably not significantly greater than about 2.5. Capsule wall porosities (determined by mercury porisimetry) of at least 3% can be obtained when the wax content is at least 10% by weight.

Typical preferred waxes have a density ranging from about 0.9 to about 1.05, melting points ranging from about 50° to about 90° C., molecular weights ranging from about 300 to about 1500, preferably about 400 to about 800, and fractional volume contractions ($V_z$) of at least about 20%. The wax component thus has a rate or extent of contraction slightly greater than the preferred polyolefins, and considerably greater than the amorphous thermoplastic resins; hence the wax contraction can be significantly greater than the contraction of a polyolefin/amorphous resin phase. Data on suitable commercial paraffin grade waxes ("Shellwax" 100, 200, 300, and 700, available from Shell Chemical Company) are set forth below:

| Grade ("Shellwax") | Melting Pt. (°C) | Density | Vz |
|---|---|---|---|
| 100 | 51.4 | 0.91 | 0.260 |
| 200 | 60.8 | 0.92 | 0.257 |
| 300 | 70.6 | 0.93 | 0.250 |
| 400 | 83.9 | 0.94 | 0.252 |

It is within the scope of this invention to add high boiling solvents and/or plasticizers to the capsule wall-forming composition. The plasticizer or flexibilizer materials lower the melt viscosity of a capsule wall-forming composition and increase capsule flexibility, thus resistance to breakage. Among the suitable plasticizers are: mineral oil, soya oil, peanut oil, and safflower oil. Anti-oxidants include octadecyl 3-(3',5'-di-t-butyl-4'-hydroxyphenyl)propionate and other compounds containing sterically hindered phenolic hydroxyls.

In general, the capsules are formed and filled by forcing a jet of fill liquid through a body of molten capsule shell material, the jet being directed to follow a desired trajectory which causes a concentric shell to form around the liquid fill material. Cooling the molten capsule shell causes it to solidify and form capsules containing liquid fill solution, the capsules being non-tacky and dry on their exterior.

In another embodiment controlled release of 4-PB as described herein is achieved by the use of an osmotic

pump. An osmotic pump comprises a shell defining an interior compartment and having an outlet passing through the shell. The interior compartment contains the active pharmaceutical ingredient. Generally the active pharmaceutical ingredient is mixed with excipients or other compositions such as a polyalkylene. The shell is generally made, at least in part, from a material (such as cellulose acetate) permeable to the liquid of the environment where the pump will be used, usually stomach acid. Once ingested, the pump operates when liquid diffuses through the shell of the pump. The liquid dissolves the composition to produce a saturated situation. As more liquid diffuses into the pump, the saturated solution containing the pharmaceutical is expelled from the pump through the outlet. This produces a nearly constant release of active ingredient, in the present case, 4-PB.

In yet another embodiment, a core comprising 4-PB or 4-PB salt is coated with a controlled release film which comprises a water insoluble material. The film can be applied by spraying an aqueous dispersion of the water insoluble material onto the core. Suitable water insoluble materials include alkyl celluloses, acrylic polymers, waxes (alone or in admixture with fatty alcohols), shellac and zein. The aqueous dispersions of alkyl celluloses and acrylic polymers preferably contain a plasticizer such as triethyl citrate, dibutyl phthalate, propylene glycol, and polyethylene glycol. The film coat can contain a water-soluble material such as polyvinylpyrrolidone (PVP) or hydroxypropylmethylcellulose (HPMC).

The core can be a granule made, for example, by wet granulation of mixed powders of 4-PB or 4-PB salt and a binding agent such as HPMC, or by coating an inert bead with 4-PB or 4-PB salt and a binding agent such as HPMC, or by spheronising mixed powders of 4-PB or 4-PB salt and a spheronising agent such as microcrystalline cellulose. The core can be a tablet made by compressing such granules or by compressing a powder comprising 4-PB.

The *in vitro* and *in vivo* release characteristics of the controlled release oral dosage forms, e.g. tablets, capsules, films, osmotic pumps etc. as described herein, can be modified by employing appropriate techniques in the art (e.g. using mixtures of different water insoluble and water soluble materials, using different plasticizers, varying the thickness of a controlled release film, including release-modifying agents in the coating, or by providing passageways through the coating etc).

Importantly, regarding the controlled release oral dosage forms of the invention as described herein, it is important in the present invention that appropriate blood plasma levels of 4-PB are achieved and maintained for sufficient time to provide the desired effect to a patient for a period of 12 to 24 hours, preferably 12 to 36h, more preferably 12 to 48h.

When administered orally to humans, an effective controlled release oral dosage form of 4-PBas described herein should exhibit the following *in vivo* characteristics: (a) peak plasma level of 4-PB occurs within about 1 to about 8 hours after administration; (b) relative 4-PB bioavailability is in the range of about 0.5 to about 1.5 compared to an orally-administered aqueous solution of 4-PB. Of course, there is variation of these parameters among subjects, depending on the size and weight of the individual subject, the subject's age, individual metabolism differences, and other factors. Indeed, the parameters may vary in an individual from day to day.

The amount of 4-PB included in the tablets, capsules, pills, films, osmotic pumps or any other solid pharmaceutical dosage form for oral administration may contain from 12.5 to 1500mg (expressed as the free acid), preferably from 12.5 to 1500mg of 4-phenylbutyric acid, e.g. 12.5, 25, 50, 75, 100, 125, 150, 200, 250, 300, 400, 500, 600, 700, 800, 900, 1000, 1200, 1400, or 1500mg 4-PB per dosage unit. Typically the amount of 4-phenylbutyric acid may be in the range from 25 to 1500 mg, suitably from 100 to 1500mg, preferred from 200 to 1500mg, 400 to 1500mg, or 600 to 1500mg per dosage unit. Suitably 4-PB may be used in a weight proportion of the overall solid dosage form, such as a compacted tablet, similar to that in which it is conventionally used.

For example 4-PB may comprise 0.5 to 95 wt%, e.g. 5-90 wt %, 15-90 wt %, 25-85 wt %, 35-85 wt %, or 50 to 80 wt% of the solid dosage form such as a compacted tablet. It is generally desirable to limit the weight of a pharmaceutical dosage from intended for swallowing by a patient to a reasonable size to facilitate swallowing, e.g around 1-2 g maximum per tablet, 1.5 g generally being a comfortable maximum in practice. Consequently the total quantity of 4-PB included in a single tablet may suitably be around 12.5 to 1500mg (expressed as the free acid), preferably from 12.5 to 1500mg of 4-phenylbutyric acid, e.g. 12.5, 25, 50, 75, 100, 125, 150, 200, 250, 300, 400, 500, 600, 700, 800, 900, 1000, 1200, 1400, or 1500mg. Suitably the quantity of the pharmaceutically acceptable organic acid and/or a pharmaceutically acceptable salt-like derivative thereof or components which can react together to form such a salt-like derivative may comprise 0.5 - 50 wt% of the solid dosage form such as a compacted tablet, for example 10 - 50 wt%.

Alternatively the formulation may be provided as a dry product as described herein ((crystalline) powder, lyophilisate, etc.) for reconstitution as a liquid formulation before use.

Formulations suitable for non-oral transmucosal administration include liquids, solutions (e.g., aqueous, non-aqueous), suspensions (e.g., aqueous, non-aqueous), emulsions (e.g., oil-in-water, water-in-oil), suppositories, pessaries, gels, pastes, ointments, creams, lotions, oils, as well as patches, adhesive plasters, depots, and reservoirs.

Formulations suitable for transdermal administration include gels, pastes, ointments, creams, lotions, and oils, as well as patches, adhesive plasters, bandages, dressings, depots, and reservoirs.

The topical and mucosa dosage forms of the present invention comprise, but are not particularly limited to, sprays, aerosols, solutions, emulsions, suspensions, eye drops or other ophthalmological formulations, and the other forms known to those skilled in the art to which the present invention belongs. See Remington's Pharmaceutical Sciences,

16th and 18th eds. Mack Publishing, Easton PA (1980 & 1990); and Introduction to Pharmaceutical Dosage Forms, 4th ed., Lea & Febiger, Philadelphia (1985). The dosage form suitable for treatment of oral mucosal tissues may be formulated as a mouthwashes or oral gels. Suitable additives (i.e. carrier and vehicle) and other substances that may be used to provide the topical and mucosa dosage forms encompassed in the present invention have been widely known to those skilled in the field of pharmacology, and depends on certain tissues to which the given pharmaceutical composition or its dosage form may be applied. Based on the above facts, the typical additives used to form a solution, an emulsion or a gel comprise, but are not particularly limited to, water, acetone, ethanol, ethylene glycol, propylene glycol, butane-1, 3-diol, isopropyl myristate, isopropyl palmitate, mineral oil and mixture thereof, and they are non-toxic and pharmaceutically available. A moisturizer or a humectant may also be added to the pharmaceutical composition and its dosage form, when desirable. Examples of the additives have been known to those skilled in the art to which the present invention belongs. For example, see Remington's Pharmaceutical Sciences, 16th and 18th eds., Mack Publishing, Easton PA (1980 & 1990). pH of the pharmaceutical composition or its dosage form may be adjusted to improve the transfer of at least one active component. Similarly, polarity, ion strength, or osmotic pressure of a solvent carrier may also be adjusted to improve the transfer of the active component.

Ointments are typically prepared from the compound and a paraffinic or a water-miscible ointment base.

Creams are typically prepared from the compound and an oil-in-water cream base. If desired, the aqueous phase of the cream base may include, for example, at least about 30% w/w of a polyhydric alcohol, i.e., an alcohol having two or more hydroxyl groups such as propylene glycol, butane-1,3-diol, mannitol, sorbitol, glycerol and polyethylene glycol and mixtures thereof. The topical formulations may desirably include a compound which enhances absorption or penetration of the compound through the skin or other affected areas. Examples of such dermal penetration enhancers include dimethylsulfoxide and related analogues.

Emulsions are typically prepared from the compound and an oily phase, which may optionally comprise merely an emulsifier (otherwise known as an emulgent), or it may comprises a mixture of at least one emulsifier with a fat or an oil or with both a fat and an oil. Preferably, a hydrophilic emulsifier is included together with a lipophilic emulsifier which acts as a stabiliser. It is also preferred to include both an oil and a fat. Together, the emulsifier(s) with or without stabiliser(s) make up the so-called emulsifying wax, and the wax together with the oil and/or fat make up the so-called emulsifying ointment base which forms the oily dispersed phase of the cream formulations.

Suitable emulgents and emulsion stabilisers include Tween 60, Span 80, cetostearyl alcohol, myristyl alcohol, glyceryl monostearate and sodium lauryl sulfate. The choice of suitable oils or fats for the formulation is based on achieving the desired cosmetic properties; since the solubility of the compound in most oils likely to be used in pharmaceutical emulsion formulations may be very low. Thus the cream should preferably be a non-greasy, non-staining and washabie product with suitable consistency to avoid leakage from tubes or other containers. Straight or branched chain, mono- or dibasic alkyl esters such as di-isoadipate, isocetyl stearate, propylene glycol diester of coconut fatty acids, isopropyl myristate, decyl oleate, isopropyl palmitate, butyl stearate, 2-ethylhexyl palmitate or a blend of branched chain esters known as Crodamol CAP may be used, the last three being preferred esters. These may be used alone or in combination depending on the properties required. Alternatively, high melting point lipids such as white soft paraffin and/or liquid paraffin or other mineral oils can be used.

Formulations suitable for intranasal administration, where the carrier is a liquid, include, for example, nasal spray, nasal drops, or by aerosol administration by nebuliser, include aqueous or oily solutions of the compound.

Formulations suitable for intranasal administration, where the carrier is a solid, include, for example, those presented as a coarse powder having a particle size, for example, in the range of about 20 to about 500 microns which is administered in the manner in which snuff is taken, i.e., by rapid inhalation through the nasal passage from a container of the powder held close up to the nose.

Formulations suitable for pulmonary administration (e.g., by inhalation or insufflation therapy) include those presented as an aerosol spray from a pressurised pack, with the use of a suitable propellant, such as dichlorodifluoromethane, trichlorofluoromethane, dichoro-tetrafluoroethane, carbon dioxide, or other suitable gases.

Formulations suitable for ocular administration include eye drops wherein the compound is dissolved or suspended in a suitable carrier, especially an aqueous solvent for the compound.

Formulations suitable for rectal administration may be presented as a suppository with a suitable base comprising, for example, natural or hardened oils, waxes, fats, semi-liquid or liquid polyols, for example, cocoa butter or a salicylate; or as a solution or suspension for treatment by enema.

Formulations suitable for vaginal administration may be presented as pessaries, tampons, creams, gels, pastes, foams or spray formulations containing in addition to the compound, such carriers as are known in the art to be appropriate.

Formulations suitable for parenteral administration (e.g., by injection), include aqueous or non-aqueous, isotonic, pyrogen-free, sterile liquids (e.g., solutions, suspensions), in which the compound is dissolved, suspended, or otherwise provided (e.g., in a liposome or other microparticulate). Such liquids may additional contain other pharmaceutically acceptable ingredients, such as anti-oxidants, buffers, preservatives, stabilisers, bacteriostats, suspending agents, thickening agents, and solutes which render the formulation isotonic with the blood (or other relevant bodily fluid)

of the intended recipient. Examples of excipients include, for example, water, alcohols, polyols, glycerol, vegetable oils, and the like. Examples of suitable isotonic carriers for use in such formulations include Sodium Chloride Injection, Ringer's Solution, or Lactated Ringer's Injection. The formulations may be presented in unit-dose or multi-dose sealed containers, for example, ampoules and vials, and may be stored in a freeze-dried (lyophilised) condition requiring only the addition of the sterile liquid carrier, for example water for injections, immediately prior to use. Extemporaneous injection solutions and suspensions may be prepared from sterile powders, granules, and tablets.

The parenteral dosage form may be administered to patients through various routes comprising, but is not particularly limited to, subcutaneous, intravenous (including a bolus injection), intramuscular, and intraarterial routes. Since such administration generally detours the natural protection of the patients from contamination sources, the parenteral dosage form should be sterile, or be sterilized before the administration to the patients. Examples of the parenteral dosage form includes, but are not particularly limited to, an injection solution, a dry product that may be easily dissolved or suspended into a pharmaceutically available vehicle for injection, an injectable suspension, and an emulsion. The suitable vehicle that may be used to provide the parenteral dosage form of the present invention is well known to those skilled in the art to which the present invention belongs. Examples of the suitable vehicle comprise, but are not particularly limited to, an aqueous vehicle including, but are not particularly limited to, USP injectable distilled water; sodium chloride injection solution, Ringer's injection solution, dextrose injection solution, dextrose and sodium chloride injection solution, and lactated Ringer's injection solution; a water-miscible vehicle including, but are not particularly limited to, ethyl alcohol, polyethylene glycol and polypropylene glycol; and a non-aqueous vehicle including, but are not particularly limited to, corn oil, cotton seed oil, peanut oil, sesame seed oil, ethyl oleate, isopropyl myristate and benzyl benzoate.

The parenteral dosage form of the present invention may also comprise at least one compound that may enhance the solubility of the active components disclosed in this specification.

In a preferred embodiment the parenteral dosage form is a sterile injection solution.

According to a preferred embodiment the sterile injection solution comprises 1.25 to 1500mg 4-PB /milliliter (expressed as the free acid), preferably from 12.5 to 1500mg 4-PB/milliliter, e.g. 12.5, 25, 50, 75, 100, 125, 150, 200, 250, 300, 400, 500, 600, 700, 800, 900, 1000, 1200, 1400, or 1500mg 4-PB/milliliter. Typically the amount of 4-PB may be in the range from 25 to 1500 mg 4-PB/milliliter, suitably from 100 to 1500mg 4-PB/milliliter, preferred from 200 to 1500mg 4-PB/milliliter, 400 to 1500mg, or 600 to 1500mg/milliliter.

Alternatively such sterile injection solution comprises 4-PB at a level of at least 0,0001% w/v. The composition can contain 0,0001-50%) w/v 4-PB. Preferably composition contains 0.005 to 40 w/v%, e.g. 0.05-35 w/v %, 0.5-30 w/v %, 0.7-30 w/v %, 1-25 w/v %, 1.5-30 w/v %, 2-25 w/v %, 2.5-25 w/v % 4-PB.

In a preferred embodiment the sterile injection solution comprise 0,5 w/v %, 1 w/v %, 2 w/v %, 3 w/v %, 4 w/v %, 5 w/v, 6 w/v % or 7 w/v % 4-PB.

According to a preferred embodiment any of the non-solid or semi-solid pharmaceutical formulations described herein, e.g. pharmaceutical formulations in the form of liquids, solutions (e.g., aqueous, non-aqueous), suspensions (e.g., aqueous, non-aqueous), emulsions (e.g., oil-in-water, water-in-oil), elixirs, drops, syrups, electuaries, mouthwashes, boluses, oils, foams, sprays, mists, or aerosols lotions, tinctures, gels, pastes, ointments, creams and the like may contain 1.25 to 1500mg 4-PB/milliliter (expressed as the free acid), preferably from 12.5 to 1500mg 4-PB/milliliter, e.g. 12.5, 25, 50, 75, 100, 125, 150, 200, 250, 300, 400, 500, 600, 700, 800, 900, 1000, 1200, 1400, or 1500mg 4-PB/milliliter. Typically the amount of 4-PB may be in the range from 25 to 1500 mg 4-PB/milliliter, suitably from 100 to 1500mg 4-PB/milliliter, preferred from 200 to 1500mg 4-PB/milliliter, 400 to 1500mg, or 600 to 1500mg 4-PB/milliliter.

Alternatively such non-solid or semi-solid pharmaceutical formulations may contain 4-PB at a level of at least 0,0001% w/v. The composition can contain 0,0001-50%) w/v 4-PB. Preferably composition contains 0.005 to 40 w/v%, e.g. 0.05-35 w/v %, 0.5-30 w/v %,0.7-30 w/v %,1-25 w/v %, 1.5-30 w/v %, 2-25 w/v %, 2.5-25 w/v % 4-PB.

In a preferred embodiment the liquid pharmaceutical compositions comprise 0,5 w/v %, 1 w/v %, 2 w/v %, 3 w/v %, 4 w/v %, 5 w/v %, 6 w/v % or 7 w/v % 4-PB.

According to a most preferred embodiment any of the solid pharmaceutical unit dosage forms of the invention as described herein, e.g. tablets (including, e.g., coated tablets), granules, powders, lozenges, pastilles, capsules (including, e.g., hard and soft gelatin capsules), cachets, pills, ampoules, suppositories, pessaries, patch, adhesive plaster, bandage, dressing, or the like comprises 12.5 to 1000mg 4-PB (expressed as the free acid), preferably from 12.5 to 1500mg of 4-PB, e.g. 12.5, 25, 50, 75, 100, 125, 150, 200, 250, 300, 400, 500, 600, 700, 800, 900, 1000, 1200, 1400, or 1500mg 4-PB. Typically the amount of 4-PB may be in the range from 25 to 1500 mg, suitably from 100 to 1500mg, preferred from 200 to 1500mg, 400 to 1500mg, or 600 to 1500mg per dosage unit.

In another preferred embodiment, the solid pharmaceutical unit dosage form contains 100mg of 4-phenylbutyric acid.

In another preferred embodiment, the solid pharmaceutical unit dosage form contains 200mg of 4-phenylbutyric acid.

In yet another preferred embodiment, the solid pharmaceutical unit dosage form contains 300mg of 4-phenylbutyric acid.

In yet another preferred embodiment, the solid pharmaceutical unit dosage form contains 400mg of 4-phenylbutyric acid.

In yet another preferred embodiment, the solid pharmaceutical unit dosage form contains 500mg of 4-phenylbutyric acid.

In another preferred embodiment, the solid pharmaceutical unit dosage form contains 600mg of 4-phenylbutyric acid.

In another preferred embodiment, the solid pharmaceutical unit dosage form contains 700mg of 4-phenylbutyric acid.

In yet another preferred embodiment, the solid pharmaceutical unit dosage form contains 800mg of 4-phenylbutyric acid.

In yet another preferred embodiment, the solid pharmaceutical unit dosage form contains 900mg of 4-phenylbutyric acid.

In yet another preferred embodiment, the solid pharmaceutical unit dosage form contains 1000mg of 4-phenylbutyric acid.

In yet another preferred embodiment, the solid pharmaceutical unit dosage form contains 1200mg of 4-phenylbutyric acid.

In yet another preferred embodiment, the solid pharmaceutical unit dosage form contains 1400mg of 4-phenylbutyric acid.

In yet another preferred embodiment, the solid pharmaceutical unit dosage form contains 1500mg of 4-phenylbutyric acid.

The present invention, in another aspect refers to a method of treatment of cancer, by providing to a patient in need thereof a sufficient amount of 4-PB as a free acid or as a salt or a hydrate or solvate thereof; in neutral form, as an isomer, as an ester; or any hydrate or solvate thereof.

In a preferred embodiment the method of treatment of cancer is characterized by providing to a patient in need thereof (a) pharmaceutical dosage unit form(s), a pharmaceutical composition, a pharmaceutical formulation, a pharmaceutical preparation, and/or a medicament comprising those as described herein.

The patient may include mammals, preferably, human, monkey, rodents (mouse, rate, and the like), and the like, and particularly, it may include any mammals, for example, human having a disease or condition related to cancer.

The term "administered" means administration of a therapeutically effective dose of the aforementioned compositions. By "therapeutically effective amount" is meant a dose that produces the effects for which it is administered. The exact dose will depend on the purpose of the treatment, and will be ascertainable by one skilled in the art using known techniques. As is known in the art and described above, adjustments for systemic versus localized delivery, age, body weight, general health, sex, diet, time of administration, drug interaction and the severity of the condition may be necessary, and will be ascertainable with routine experimentation by those skilled in the art.

The cancer that may be treated according to the present invention may be at least one selected from the group consisting of Acute myeloid leukemia, Adrenocortical carcinoma, AIDS-related cancers, AIDS-related lymphoma, Anal cancer, Appendix cancer, Astrocytoma, childhood cerebellar or cerebral, Basal cell carcinoma, Bile duct cancer, extrahepatic Bladder cancer, Bone cancer, Osteosarcoma/Malignant fibrous histiocytoma, Brainstem glioma, Brain cancer, Brain tumor, cerebellar astrocytoma, cerebral astrocytoma/malignant glioma, Ependymoma, medulloblastoma, supratentorial primitive neuroectodermal tumors, hypothalamic glioma, Breast cancer, Bronchial adenomas/carcinoids, Burkitt lymphoma, gastrointestinal tumor, lymphoma, primary Cerebellar astrocytoma, childhood cerebral astrocytoma/Malignant glioma, Cervical cancer, Childhood cancers, Chronic lymphocytic leukemia, Chronic myelogenous leukemia, Chronic myeloproliferative disorders, Colon Cancer, Cutaneous T-cell lymphoma, Desmoplastic small round cell tumor, Endometrial cancer, Ependymoma, Esophageal cancer, Ewing's sarcoma, Extracranial germ cell tumor, Extragonadal Germ cell tumor, Extrahepatic bile duct cancer, Intraocular melanoma, Retinoblastoma, Gallbladder cancer, Gastric (Stomach) cancer, Gastrointestinal Tumor, Gastrointestinal stromal tumor (GIST), Germ cell tumor, extracranial, extragonadal, or ovarian tumor, Gestational trophoblastic tumor, Glioma of the brain stem, Glioma, Childhood Cerebral Astrocytoma, Glioma, Childhood Visual Pathway and Hypothalamic, Gastric carcinoid, Hairy cell leukemia, Head and neck cancer, Heart cancer, Hepatocellular (liver) cancer, Hodgkin lymphoma, Hypopharyngeal cancer, Hypothalamic and visual pathway glioma, Intraocular Melanoma, Islet Cell Carcinoma, Kaposi sarcoma, Kidney cancer (renal cell cancer), Laryngeal Cancer, Leukemias, acute lymphoblastic leukemia (acute lymphocytic leukemia), acute myeloid leukemia (acute myelogenous leukemia), chronic lymphocytic leukemia (chronic lymphocytic leukemia), chronic myelogenous leukemia (chronic myeloid leukemia), hairy cell leukemia, liposarcoma, Liver Cancer, Non-Small Cell Lung Cancer, Small Cell Lung Cancer, Lymphoma, AIDS-related Lymphoma, Burkitt Lymphoma, cutaneous T-Cell Lymphoma, Hodgkin Lymphoma, Non-Hodgkin Lymphoma, Primary Central Nervous System Lymphoma, Macroglobulinemia, Waldenström Macroglobulinemia, Malignant Fibrous Histiocytoma of Bone/Osteosarcoma, Medulloblastoma, Childhood Melanoma, Melanoma, Intraocular Merkel Cell Carcinoma, Adult Malignant Mesothelioma, Childhood Mesothelioma,

Metastatic Squamous Neck Cancer with Occult Primary, Mouth Cancer, Multiple Endocrine Neoplasia Syndrome, Multiple Myeloma/Plasma Cell Neoplasm, Mycosis Fungoides, Myelodysplastic Syndromes, Myelodysplastic/Myeloproliferative Diseases, Myelogenous Leukemia, Chronic Myeloid Leukemia, Adult Acute Myeloid Leukemia, Childhood Acute Myeloma, Multiple (Cancer of the Bone-Marrow), Myeloproliferative Disorders, Nasal cavity and paranasal sinus cancer, Nasopharyngeal carcinoma, Neuroblastoma, Non-Hodgkin lymphoma, Non-small cell lung cancer, Oral Cancer, Oropharyngeal cancer, Osteosarcoma/malignant fibrous histiocytoma of bone, Ovarian cancer, Ovarian epithelial cancer (Surface epithelial-stromal tumor), Ovarian germ cell tumor, Ovarian low malignant potential tumor, Pancreatic cancer, Pancreatic islet cell cancer, Parathyroid cancer, Penile cancer, Pharyngeal cancer, Pheochromocytoma, Pineal astrocytoma, Pineal germinoma, Pineoblastoma and supratentorial primitive neuroectodermal tumors, Pituitary adenoma, Plasma cell neoplasia/Multiple mieloma, Pleuropulmonary blastoma, Primary central nervous system lymphoma, Prostate cancer, Rectal cancer, Renal cell carcinoma (kidney cancer), Renal pelvis and ureter cancer, transitional cell cancer, Retinoblastoma, Rhabdomyosarcoma, Salivary gland cancer, Sarcoma, soft tissue Sarcoma, uterine Sarcoma, Sézary syndrome, Skin cancer (nonmelanoma), Skin cancer (melanoma), Skin carcinoma (Merkel cell), Small cell lung cancer, Small intestine cancer, Squamous cell carcinoma, Squamous neck cancer with occult primary, stomach (gastric) cancer, Supratentorial primitive neuroectodermal tumor, T-Cell lymphoma, Testicular cancer, Throat cancer, Thymoma, Thymic carcinoma, Thyroid cancer, Trophoblastic tumor, Urethral cancer, Uterine cancer, Uterine sarcoma, Vaginal cancer, hypothalamic glioma, Vulvar cancer, Waldenström macroglobulinemia, Wilms tumor (kidney cancer).

In a very preferred embodiment the cancer is prostate cancer.

According to one specific embodiment of the present invention, the cancer is metastatic. According to another embodiment of the present invention, the cancer is characterized in that it is intractable or resistant to the chemotherapy or radiotherapy.

In a preferred embodiment the pharmaceutical dosage unit forms of this invention can be administered as the sole pharmaceutical dosage unit forms or in combination with one or more other pharmaceutical agents where the combination causes no unacceptable adverse effects. The present invention relates also to such combinations. For example, the compounds of this invention can be combined with known anti-hyper-proliferative or other indication agents, and the like, as well as with admixtures and combinations thereof. Other indication agents include, but are not limited to, anti-angiogenic agents, mitotic inhibitors, alkylating agents, antimetabolites, DNA-intercalating antibiotics, growth factor inhibitors, cell cycle inhibitors, enzyme inhibitors, toposisomerase inhibitors, biological response modifiers, or anti-hormones.

The additional pharmaceutical agent can be afinitor, aldesleukin, alendronic acid, alfaferone, alitretinoin, allopurinol, aloprim, aloxi, altretamine, aminoglutethimide, amifostine, amrubicin, amsacrine, anastrozole, anzmet, aranesp, arglabin, arsenic trioxide, aromasin, 5-azacytidine, azathioprine, BAY 80-6946, BCG or tice BCG, bestatin, betamethasone acetate, betamethasone sodium phosphate, bexarotene, bleomycin sulfate, broxuridine , bortezomib, busulfan, calcitonin, campath, capecitabine, carboplatin, casodex, cefesone, celmoleukin, cerubidine, chlorambucil, cisplatin, cladribine, cladribine, clodronic acid, cyclophosphamide, cytarabine, dacarbazine, dactinomycin, DaunoXome, decadron, decadron phosphate, delestrogen, denileukin diftitox, depo-medrol, deslorelin, dexrazoxane, diethylstilbestrol, diflucan, docetaxel, doxifluridine, doxorubicin, dronabinol, DW-166HC, eligard, elitek, ellence, emend, epirubicin, epoetin alfa, epogen, eptaplatin, ergamisol, estrace, estradiol, estramustine phosphate sodium, ethinyl estradiol, ethyol, etidronic acid, etopophos, etoposide, fadrozole, farston, filgrastim, finasteride, filgrastim, floxuridine, fluconazole, fludarabine, 5-fluorodeoxyuridine monophosphate, 5-fluorouracil (5-FU), fluoxymesterone, flutamide, formestane, fosteabine, fotemustine, fulvestrant, gammagard, gemcitabine, gemtuzumab, gleevec, gliadel, goserelin, granisetron HCl, histrelin, hycamtin, hydrocortone, eyrthro-hydroxynonyladenine, hydroxyurea, ibritumomab tiuxetan, idarubicin, ifosfamide, interferon alpha, interferon-alpha 2, interferon alfa-2A, interferon alfa-2B, interferon alfa-n1 , interferon alfa-n3, interferon beta, interferon gamma-1 a, interleukin-2, intron A, iressa, irinotecan, kytril, lentinan sulfate, letrozole, leucovorin, leuprolide, leuprolide acetate, lapatinib, levamisole, levofolinic acid calcium salt, levothroid, levoxyl, lomustine, lonidamine, marinol, mechlorethamine, mecobalamin, medroxyprogesterone acetate, megestrol acetate, melphalan, menest, 6-mercaptopurine, Mesna, methotrexate, metvix, miltefosine, minocycline, mitomycin C, mitotane, mitoxantrone, Modrenal, Myocet, nedaplatin, neulasta, neumega, neupogen, nilutamide, nolvadex, NSC-631570, OCT-43, octreotide, ondansetron HCl, orapred, oxaliplatin, paclitaxel, pediapred, pegaspargase, Pegasys, pentostatin, picibanil, pilocarpine HCl, pirarubicin, plicamycin, porfimer sodium, prednimustine, prednisolone, prednisone, premarin, procarbazine, procrit, raltitrexed, RDEA 119, rebif, rhenium-186 etidronate, rituximab, roferon-A, romurtide, salagen, sandostatin, sargramostim, semustine, sizofiran, sobuzoxane, solu-medrol, sparfosic acid, stem-cell therapy, streptozocin, strontium-89 chloride, sunitinib, synthroid, tamoxifen, tamsulosin, tasonermin, tastolactone, taxotere, teceleukin, temozolomide, teniposide, testosterone propionate, testred, thioguanine, thiotepa, thyrotropin, tiludronic acid, topotecan, toremifene, tositumomab, trastuzumab, treosulfan, tretinoin, trexall, trimethylmelamine, trimetrexate, triptorelin acetate, triptorelin pamoate, UFT, uridine, valrubicin, vesnarinone, vinblastine, vincristine, vindesine, vinorelbine, virulizin, zinecard, zinostatin stimalamer, zofran, ABI-007, acolbifene, actimmune, affinitak, aminopterin, arzoxifene, asoprisnil, atamestane, atrasentan, sorafenib (BAY 43-9006), avastin, CCI-779 , CDC-501 , celebrex, cetuximab, crisnatol, cyproterone acetate, decitabine, DN-101 , doxorubicin-MTC, DSLIM, dutasteride, edotecarin, eflornithine, exatecan, fenretinide, histamine dihydrochloride, histrelin hydrogel implant, holmium-166 DOTMP, ibandronic acid, interferon gamma, intron-PEG, ixabepilone, keyhole limpet hemocyanin, L-651582, lanreotide, lasofoxifene, libra, lonafarnib, miproxifene, minodronate, MS-209, liposomal MTP-PE, AAX-6, nafarelin, nemorubicin, neovastat, nolatrexed, oblimersen, onco-TCS, osidem, paclitaxel polyglutamate, pamidronate disodium, PN-401 , QS-21 , quazepam, R-1549, raloxifene, ranpirnase, 13-cis -retinoic acid, satraplatin, seocalcitol, T-138067, tarceva, taxoprexin, thymosin alpha 1 , tiazofurine, tipifarnib, tirapazamine, TLK-286 , toremifene, TransMI D-107R, valspodar, vapreotide, vatalanib, verteporfin, vinflunine, Z-100, zoledronic acid or combinations thereof. Optional anti-hyper-proliferative agents which can be added to the composition include but are not limited to compounds listed on the cancer chemotherapy drug regimens in the 11th Edition of the Merck Index, (1996), which is hereby incorporated by reference, such as asparaginase, bleomycin, carboplatin, carmustine, chlorambucil, cisplatin, colaspase, cyclophosphamide, cytarabine, dacarbazine, dactinomycin, daunorubicin, doxorubicin (adriamycine), epirubicin, epothilone, an epothi lone derivative, etoposide, 5-fluorouracil, hexamethylmelamine, hydroxyurea, ifosfamide, irinotecan, leucovorin, lomustine, mechlorethamine, 6-mercaptopurine, mesna, methotrexate, mitomycin C, mitoxantrone, prednisolone, prednisone, procarbazine, raloxifen, streptozocin, tamoxifen, thioguanine, topotecan, vinblastine, vincristine, and vindesine.

Other anti-hyper-proliferative agents suitable for use with the composition of the invention include but are not limited to those compounds acknowledged to be used in the treatment of neoplastic diseases in Goodman and Gilman's The Pharmacological Basis of Therapeutics (Ninth Edition), editor Molinoff et al. , publ. by McGraw-Hill, pages 1225-1287, (1996), which is hereby incorporated by reference, such as aminoglutethimide, L-asparaginase, azathioprine, 5-azacytidine cladribine, busulfan, diethylstilbestrol, 2',2'-difluorodeoxycytidine, docetaxel, 5-fluorodeoxyuridine, 5-fluorodeoxyuridine monophosphate, fludarabine phosphate, fluoxymesterone, flutamide, hydroxyprogesterone caproate, idarubicin, interferon, medroxyprogesterone acetate, megestrol acetate, melphalan, mitotane, paclitaxel, pentostatin, N-phosphonoacetyl-L-aspartate (PALA), plicamycin, semustine, teniposide, testosterone propionate, thiotepa, trimethylmelamine, uridine, and vinorelbine.

Other anti-hyper-proliferative agents suitable for use with the composition of the invention include but are not limited to other anti-cancer agents such as epothilone and its derivatives, irinotecan, raloxifen and topotecan.

The active agents or compounds according to the present invention may be administered alone or in combination with other treatments. In a preferred embodiment the pharmaceutical composition of the present invention may be administered in combination with an anti-hormone treatment, e.g. an anti-androgen treatment.

In another preferred embodiment the pharmaceutical dosage unit forms of the invention can be administered in combination with one or more anti-cancer treatments such as hypothermia, hyperthermia, or x-ray radiation.

The concentration of the active ingredients or compounds of a pharmaceutical composition according to the present invention may be further adjusted to the intended dosage regimen, the intended usage duration, the exact amount and ratio of all ingredients of the composition and further factors and parameter known to the person skilled in the art.

It will be appreciated by one of skill in the art that appropriate dosages of the compounds of the invention, and compositions comprising the compounds of the invention, can vary from patient to patient. Determining the optimal dosage will generally involve the balancing of the level of therapeutic benefit against any risk or deleterious side effects. The selected dosage level will depend on a variety of factors including, but not limited to, the activity of the particular compound of the invention, the route of administration, the time of administration, the rate of excretion of the compound, the duration of the treatment, other drugs, compounds, and/or materials used in combination, the severity of the condition, and the species, sex, age, weight, condition, general health, and prior medical history of the patient. The amount of compound and route of administration will ultimately be at the discretion of the physician, veterinarian, or clinician, although generally the dosage will be selected to achieve local concentrations at the site of action which achieve the desired effect without causing substantial harmful or deleterious side-effects.

Administration can be effected in one dose, continuously or intermittently (e.g., in divided doses at appropriate intervals) throughout the course of treatment. Methods of determining the most effective means and dosage of administration are well known to those of skill in the art and will vary with the formulation used for therapy, the purpose of the therapy, the target cell(s) being treated, and the subject being treated. Single or multiple administrations can be carried out with the dose level and pattern being selected by the treating physician, veterinarian, or clinician.

For the dosage forms may be varied according to the present invention, the composition, shape, and type of the dosage may be widely varied according to their use. For example, the dosage form used to treat acute diseases may further comprises at least one active component in a larger amount than that of the dosage form used to treat the same chronic diseases. Similarly, the parenteral dosage form may further comprise at least one active component in a smaller amount than the oral dosage form used to treat the same diseases. These and other modes of the certain dosage form included in the present invention are very widely varied, and they are evident to those skilled in the art to which the present invention belongs. For example, see Remington's Pharmaceutical Sciences, 18th ed., Mack Publishing, Easton PA (1990).

According to one aspect of the invention, a suitable daily dose of 4-PB is in the range of about 10 μg to about 250 mg (more typically about 100 μg to about 25 mg) per kilogram body weight of the subject per day. Where 4-PB is in the form of a salt, a derivative, an ester, an amide, a prodrug, or the like, as described herein, the amount administered is calculated on the basis of the parent compound and so the actual weight to be used is increased proportionately. The dose may be administered once or several times a day with the daily effective range. Generally, children should be administered in a lesser dose according to the weight and age of the children.
In a preferred embodiment the dose is administered once a day.
In another preferred embodiment the dose is administered twice a day.
In another preferred embodiment the dose is administered every 12 hours.
In another preferred embodiment the dose is administered thrice a day.
In another preferred embodiment the dose is administered every 8 hours.
In yet another preferred embodiment the dose is administered four times a day.
In another preferred embodiment the dose is administered every 6 hours.
In yet another embodiment the dose is administered five times a day.

## EXAMPLES

For the preparation of the powder components for filling hard gelatine capsules it is possible to use the same powder components as those used for the preparation of tablets. The release kinetics in tablets are also dependent upon geometric factors, such as the shape and size of the tablets. Biconvex tablets having a diameter of approximately 5 - 11 mm, especially 7 - 9 mm, and a thickness of 3 - 10 mm, especially 6.8 mm, are preferred.
A preferred tablet contains, for example:

- o about 250 mg of sodium phenylbutyrate,
- o about 146 mg of hydroxypropylmethylcellulose type 2208 USP XXII of 100 or 4000 cps,
- o about 261 mg of lactose,
- o about 30 to 60 mg, in particular 31.25 mg, of microcrystalline cellulose (Avicel® PH 102, Select chemie, or Cellactose(R), Meggle),
- o about 10 mg of hardened vegetable oil, or e.g. talcum,
- o about 1.5 mg of magnesium stearate, and
- o optionally about 1 mg of highly dispersed silicon dioxide, so that cores prepared thereof weigh about 0.7 g.

Advantageously, a number of 24,000 of the cores are provided with a film coating for example by using a colloidal dispersion containing about 7,850 g of isopropylalcohol, about 3,360 g of Eudragit ®L 12.5, about 66 g of dibutyl phthalat, about 18.0 g of Miglyol® 812 and about 56 g of polyethylenglycol PEG 400.
For tablets containing 100 and 500 mg of active ingredient, corresponding aliquots of the excipients should be used.
Preferred pharmaceutical dosage forms contain 250 mg of sodium 4-phenylbutyrate, and are specifically the tablets described in Example 1.
In a further embodiment the invention relates also to a process for the preparation of a pharmaceutical dosage form according to the above description, characterised in that the dosage form is prepared in a conventional manner.
The constituents of the tablet cores are, if necessary, ground to the desired particle size, mixed homogeneously with one another at the same time or in a specific sequence and, optionally, granulated by moistening with water, dispersing and drying the granular mass. If the mixture is granulated, the fillers, flow agents and lubricants can be added to the granules after granulation. The mixture of the core constituents is compressed to form tablets having a hardness of approximately 50 - 100 N, preferably 90 N, or may be introduced as such into hard gelatine capsules.
The film-coating is effected in a conventional manner by mixing the constituents of the film coating with water, coating the compressed tablet cores therewith and drying at approximately from 30 to 40 degrees centigrade, preferably approximately 35 degrees centigrade.
In a further embodiment the invention relates to the use of a pharmaceutical dosage form in accordance with the present invention for the treatment of diseases that can be influenced by said dosage form, comprising the once or twice daily oral administration of a pharmaceutical dosage form according to the present invention to a patient in need thereof and to be treated.
Depending upon the age and weight of the patient, the nature and severity of the illness, as well as the general condition of the patient, and also on the salt of the 4-phenylbutyric acid to be administered, the dosage forms used contain about 100 to about 1,000 mg, preferably 250 mg of active ingredient.
Diseases that can be beneficially influenced by the present dosage form include in particular benign prostate hyperplasy, cancer, leukemias, cystic fibrosis, AIDS, kidney and liver diseases, thalassemia and urea cycle disorders.

For example, the treatment of an adenocarcinoma of the prostate can be carried out by administering twice daily, e.g. in the morning and evening, a pharmaceutical tablet formulation containing 250 mg of sodium 4-phenylbutyrate. The therapeutic effect is evident after treatment of about one month when the pain disappears. Treatment may be continued for several month or years until a satisfactory effect is observed or the patient is total free of the disease. The 4-phenylbutyrate exerts no side effects even over a long period of treatment.

The following Examples illustrate the invention but do not constitute a limitation thereof.

**Example 1:** Production of Slow Release Tablet with 250 mg of Sodium 4-Phenylbutyrate

A mixture of 6,000.0 g of sodium 4-phenylbutyrate from Triple Crown America, Inc., 6,280.0 g of lactosum monohydricum 3,500.0 g of Methocel K100 M Premium (Prochem), and 750.0 g of Avicel PH 102 (Select Chemie) is wettened with 4,000.0 g of aqua purificata (water purified by inversion osmosis) and dried in cold air during 18 hours. The mixture is forced through a sieve IV mm and dried again during 10 hours with air of 40 degrees centigrade A mixture of 240.0 g of talcum and 30.0 g of magnesium stearate is admixed during 20 minutes and the mixture is pressed into tablets of 0.70 g each, a thickness of about 6.8 mm and with a hardness of 90 Newton. Yield: 24,000 tablet cores.

The mixing is carried out with a Diosna Mixer, the drying in a Luekon drying cabinet, the sieving with a Koehler and Bosshard sieving machine, and the tablet pressing with a Korsch tablet press EK II.

The cores are provided with a film coating for example by using a colloidal dispersion containing 7,850 g of isopropylalcohol, 3,360 g of Eudragit L 12.5, 66 g of dibutyl phthalat, 18.0 g of Miglyol 812, and 56 g of polyethylenglycol PEG 400. The suspension is sprayed at 3.5 atue and 25 degrees centigrade onto the 24,000 cores. The film-coated tablets are dried in a circulating air drying cabinet for at least 4 hours at 35 degrees centigrade

**Example 2: Human Test Results**

Following are descriptions of treatments and test results obtained from human patients.

**Patient Nr. 1, MM, born Febr. 05, 1909:**

This patient presented on April 24, 1995 with a cancer of the prostate with a prostate specific antigen (PSA) of 36 μg/l and with positive prostate biopsies, revealing adenocarcinoma of the prostate. The positive bone scan revealed multiple bone metastasis with lumbar and thoracic spine, ribs and pelvis. Because of progressive bone pain he underwent bilateral orchiectomy in February 1996. He became free of pain until November 1998 when he again suffered bone pain mainly in his back. He was than treated orally twice daily with 250 mg of sodium phenylbutyrate retard tablets according to Example 1. Four weeks later he was free of pain. He stayed on this medication until June 2001 without any other symptoms of the prostate carcinoma. The PSA and the alkaline phosphatase were always within normal limits. No side effects attributable to the treatment have been observed.

A work-up in June 2002 revealed no symptoms of signs of an active prostate carcinoma. He stays now in complete unmaintained remission for 12 months.

**Patient Nr. 2, KR, born July 24, 1919:**

This patient presented in March 1997 with a pathological fracture of the 8th thoracic vertebra caused by metastatic destruction. The multiple biopsies of the slightly enlarged prostate were positive for adenocarcinoma on both sides. The bone scan showed multiple uptake in ribs and vertebrates. On April 10th 1997 he underwent bilateral orchidectomy. From May 1997 until September 2000 he was treated orally twice daily with 250 mg of sodium 4-phenylbutyrate retard tablets according to Example 1. The last control in August 2001 revealed no symptoms. The bone scan showed no pathological uptakes. The 4-phenylbutyrate was well tolerated without side-effects.

After 20 months in unmaintained remission he died from a secondary disease. His post mortem examination revealed histological no tumor tissue in the prostate or elsewhere.

**Patient Nr. 3, M.W., born March 11, 1911:**

In October 1997 a biopsy of the prostate due to elevated PSA (17.5 μg/l) revealed a cancer of the prostate. He was treated with a LH-RH agonist (Decapeptyl retard monthly). During 1998 his alkaline phosphatase begun to raise from 132 up to 167 U/l (normal value less than 110 U/l). Beginning in November 1998, he was treated orally twice daily with 250 mg of sodium 4-phenylbutyrate according to the retard tablets of Example 1. His PSA dropped to unmeasurable levels and his alkaline phosphatase dropped gradually to 114 U/l on December 14, 1999. He never complained about bone pain. He died on April 24, 2000, due to heart attack with congestive heart failure. The phenylbutyrate was well

tolerated without side-effects.

## Claims

1. A pharmaceutical unit dosage form comprising 4-phenylbutyric acid (4-PB) as a free acid or as a salt; as a prodrug, a codrug, a co-crystal or any salt or hydrate or solvate thereof.

2. The pharmaceutical unit dosage form according to claim 1, wherein said pharmaceutical unit dosage form further comprises one pharmaceutically acceptable ingredient.

3. The pharmaceutical unit dosage form according to claim 1 or 2, wherein the salt form of 4-PB is of the alkaline or the earth alkaline form.

4. The pharmaceutical unit dosage form according to claims 1 to 3, wherein the dosage form contains between 12.5 and 1500 mg of 4-PB.

5. The pharmaceutical unit dosage form according to any of the preceding claims, wherein the dosage form is prepared to provide for rapid, slow, immediate, delayed, timed or sustained release.

6. The pharmaceutical unit dosage form according to any of the preceding claims, wherein the dosage form is suitable for topical, enteral, parenteral, intradermal, intramuscular, intraperitoneal, intravenous, subcutaneous, intranasal, buccal, inhalational, epidural or oral application.

7. The pharmaceutical unit dosage form according to any of the preceding claims, wherein the dosage form is selected from the group consisting of liquid, solution, suspension, emulsion, elixir, syrup, electuary, mouthwash, drops, tablet, granule, powder, lozenge, pastille, capsule, cachet, pill, ampoule, bolus, suppository, pessary, tincture, gel, paste, ointment, cream, lotion, oil, foam, spray, mist, film, osmotic pump, bandage, dressing, depot, reservoir, injection solution or aerosol.

8. The pharmaceutical unit dosage form according to claim 7, wherein the dosage form is a controlled release capsule.

9. The pharmaceutical unit dosage form according to any of the preceding claims wherein the dosage form comprises liposomes or polymersomes.

10. The pharmaceutical unit dosage form according to any of the preceding claims, wherein the 4-PB is present in an amount of 0.5 to 7 % by weight of the unit dosage form.

11. The pharmaceutical unit dosage form according to any of the preceding claims, wherein the dosage form further comprises at least one additional pharmaceutical agent.

12. The pharmaceutical unit dosage form according to any of the preceding claims for use in the prevention or treatment of cancer.

13. The pharmaceutical unit dosage form according to claim 11, wherein the cancer to be prevented or treated is prostate cancer.

14. The pharmaceutical unit dosage form according to claim 12 or 13, wherein the dosage form is administered to a mammal, preferably a human.

15. The pharmaceutical unit dosage form according to any of the preceding claims wherein the dose is administered once, twice, thrice, four times or five times a day.

Europäisches Patentamt
European Patent Office
Office européen des brevets

# EUROPEAN SEARCH REPORT

Application Number
EP 12 00 5899

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2006/059237 A1 (LUNAMED INC [CH]; TRUOG PETER [CH]) 8 June 2006 (2006-06-08)<br>* abstract *<br>* page 1, paragraph 2 *<br>* page 16, last paragraph - page 24 *<br>* page 48, line 21 - page 51, line 10 *<br>* page 51, line 20 - page 52, line 3 *<br>* page 54, line 17 - page 56, line 5 *<br>* page 59, line 30 - page 60, line 9 *<br>* example 1 *<br>* claims 1-152 *<br>----- | 1-15 | INV.<br>A61K31/19<br>A61K9/00<br>A61P43/00<br>A61P35/00 |
| X | EP 1 291 015 A1 (LUNAMED AG [CH]) 12 March 2003 (2003-03-12)<br>* abstract *<br>* paragraph [0001] *<br>* paragraph [0014] *<br>* paragraph [0016] - paragraph [0038] *<br>* examples 1,2 *<br>* claims 1-9 *<br>----- | 1-12,14, 15 | |
| X | WO 2007/035029 A1 (SK CORP [KR]; CHO JEONG-WOO [KR]; CHOI SANG-RAK [KR]; HWANG SUN-GWAN [) 29 March 2007 (2007-03-29)<br>* abstract *<br>* paragraph [0005] *<br>* paragraph [0022] *<br>* paragraph [0043] - paragraph [0044] *<br>* claims 1-9 *<br>----- | 1-11,14, 15 | TECHNICAL FIELDS SEARCHED (IPC)<br>A61K |
| X | EP 2 389 932 A1 (LUNAMED AG [CH]) 30 November 2011 (2011-11-30)<br>* abstract *<br>* paragraph [0001] *<br>* paragraph [0016] - paragraph [0020] *<br>* example 3 *<br>* claims 1-15 *<br>-----<br>-/-- | 1-11,14, 15 | |

The present search report has been drawn up for all claims

| Place of search | Date of completion of the search | Examiner |
|---|---|---|
| The Hague | 14 November 2012 | Taylor, Mark |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 12 00 5899

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (IPC) |
| A | EP 2 397 458 A1 (LUNAMED AG [CH]) 21 December 2011 (2011-12-21) * the whole document * ----- | 1-15 | |
| A,P | Ucyclyd Pharma: "BUPHENYL (sodium phenylbutyrate) Tablets", ureacycle.com - Prescribing Information , XP002687148, Retrieved from the Internet: URL:http://pi.medicis.us/buphenyl.pdf [retrieved on 2012-11-14] * the whole document * ----- | 1-15 | |
| | | | TECHNICAL FIELDS SEARCHED (IPC) |

The present search report has been drawn up for all claims

| Place of search | Date of completion of the search | Examiner |
|---|---|---|
| The Hague | 14 November 2012 | Taylor, Mark |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

2
EPO FORM 1503 03.82 (P04C01)

## ANNEX TO THE EUROPEAN SEARCH REPORT ON EUROPEAN PATENT APPLICATION NO. EP 12 00 5899

This annex lists the patent family members relating to the patent documents cited in the above-mentioned European search report.
The members are as contained in the European Patent Office EDP file on
The European Patent Office is in no way liable for these particulars which are merely given for the purpose of information.

14-11-2012

| Patent document cited in search report | Publication date | Patent family member(s) | Publication date |
|---|---|---|---|
| WO 2006059237 A1 | 08-06-2006 | CA 2582255 A1 | 08-08-2006 |
| | | EP 1799759 A1 | 27-06-2007 |
| | | JP 2008511611 A | 17-04-2008 |
| | | US 2006045912 A1 | 02-03-2006 |
| | | WO 2006059237 A1 | 08-06-2006 |
| EP 1291015 A1 | 12-03-2003 | AT 455539 T | 15-02-2010 |
| | | AU 2002322964 B2 | 16-10-2008 |
| | | CA 2459165 A1 | 20-03-2003 |
| | | CN 1553799 A | 08-12-2004 |
| | | DK 1427396 T3 | 25-05-2010 |
| | | EP 1291015 A1 | 12-03-2003 |
| | | EP 1427396 A1 | 16-06-2004 |
| | | ES 2339536 T3 | 21-05-2010 |
| | | HK 1071847 A1 | 09-04-2010 |
| | | JP 2005508901 A | 07-04-2005 |
| | | PT 1427396 E | 14-04-2010 |
| | | US 2004180962 A1 | 16-09-2004 |
| | | WO 03022253 A1 | 20-03-2003 |
| WO 2007035029 A1 | 29-03-2007 | EP 1937241 A1 | 02-07-2008 |
| | | JP 2009508940 A | 05-03-2009 |
| | | US 2009221714 A1 | 03-09-2009 |
| | | WO 2007035029 A1 | 29-03-2007 |
| EP 2389932 A1 | 30-11-2011 | EP 2389932 A1 | 30-11-2011 |
| | | WO 2011147584 A1 | 01-12-2011 |
| | | WO 2011147585 A1 | 01-12-2011 |
| | | WO 2011147586 A1 | 01-12-2011 |
| | | WO 2011147587 A1 | 01-12-2011 |
| | | WO 2011147588 A1 | 01-12-2011 |
| EP 2397458 A1 | 21-12-2011 | EP 2397458 A1 | 21-12-2011 |
| | | WO 2011160821 A2 | 29-12-2011 |
| | | WO 2011160822 A1 | 29-12-2011 |
| | | WO 2011160823 A1 | 29-12-2011 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **BRUSILOW SW ; MAESTRI NE.** Urea cycle disorders: Diagnosis, pathophysiology, and therapy. *Adv. Pediatr.,* 1996, vol. 43, 127-170
- **LANNITTI ; PALMIERI.** *Drugs R D,* 2011, vol. 11 (3), 227-249
- Methods and Principles in Medicinal Chemistry. Wiley-VCH, 2010
- Analgesics: From Chemistry and Pharmacology to Clinical Application. Wiley-VCH, 2002, 13-126
- **FULDA.** *Drug Discovery Today,* 2010, vol. 15, 1590-1611
- **BROWN et al.** *Mol Aspects Medicine,* 2008, vol. 29, 433-452
- **ANAND et al.** *Biochem. Pharmacol.,* 2008, vol. 76, 1590-1611
- **RIOS et al.** *J. Ethnopharmacol.,* 2010, vol. 128, 1-14
- **DESIRAJU.** *CrystEngComm,* 2003, vol. 5 (82), 466-467
- **DUNITZ.** *CrystEngComm,* 2003, vol. 5 (91), 506
- **ZAWOROTKO.** *Crystal Growth & Design,* 2007, vol. 7 (1), 4-9
- Remington's Pharmaceutical Sciences. Mack Publishing Company, 1990
- Handbook of Pharmaceutical Excipients. 2005
- Kirk-Othmer Encyclopedia of Chemical Technology. John Wiley & Sons, 1966, vol. 11, 242
- Remington's Pharmaceutical Sciences. Mack Publishing, 1980
- Introduction to Pharmaceutical Dosage Forms. Lea & Febiger, 1985
- 11th Edition of the Merck Index. 1996
- Goodman and Gilman's The Pharmacological Basis of Therapeutics. McGraw-Hill, 1996, 1225-1287
- Remington's Pharmaceutical Sciences. Mack Publishing, 1990